# EUROPÄISCHE PATENTSCHRIFT

(11) **EP 2 513 188 B1**
(45) Veröffentlichungstag und Bekanntmachung des Hinweises auf die Patenterteilung: **27.04.2016**
(21) Anmeldenummer: 10796356.3
(22) Anmeldetag: 15.12.2010
(51) Int. Cl.: C08G 63/12, A01N 25/02, A01N 25/30

(54) **HYPERVERZWEIGTE POLYESTER MIT HYDROPHOBEM KERN ZUR SOLUBILISIERUNG SCHWERLÖSLICHER WIRKSTOFFE**
HYPERBRANCHED POLYESTER WITH A HYDROPHOBIC NUCLEUS FOR SOLUBILIZING POORLY SOLUBLE ACTIVE SUBSTANCES
POLYESTERS HYPERRAMIFIÉS À NOYAU HYDROPHOBE POUR LA SOLUBILISATION D'AGENTS ACTIFS PEU SOLUBLES

(30) Priorität: 18.12.2009 EP 09179828
(43) Veröffentlichungstag der Anmeldung: 24.10.2012
(73) Patentinhaber: BASF SE, 67056 Ludwigshafen am Rhein (DE)
(72) Erfinder: TÜRK, Holger, 68161 Mannheim (DE); HABERECHT, Monika, 67063 Ludwigshafen (DE); YAMADA, Hiroe, 66113 Saarbrücken (DE); BRUCHMANN, Bernd, 67251 Freinsheim (DE); SCHÖNFELDER, Daniel, B-1000 Bruxelles (BE); ISHAQUE, Michael, 68165 Mannheim (DE); TROPPMANN, Ulrike, 67105 Schifferstadt (DE); CLAUSS, Joachim, 64297 Darmstadt (DE); LIEW, Chee Chin, 67098 Bad Dürkheim (DE)
(74) Vertreter: BASF IP Association
(86) Internationale Anmeldenummer: PCT/EP2010/069680
(87) Internationale Veröffentlichungsnummer: WO 2011/073220

(56) Entgegenhaltungen:
- WO-A1-2007/125028
- WO-A2-2008/059234
- DE-A1- 19 505 100
- US-A- 3 697 428
- US-A1- 2003 118 692
- DATABASE WPI Week 200830 Thomson Scientific, London, GB; AN 2008-E48995 XP002621383, & WO 2008/013107 A1 (NISSHIN OIL MILLS LTD) 31. Januar 2008 (2008-01-31)

## Beschreibung

Gegenstand der Erfindung ist eine Zusammensetzung enthaltend einen Wirkstoff, der in Wasser bei 20 °C zu höchstens 10 g/L löslich ist, und einen hyperzverzweigten Polyester basierend auf einer hydrophoben Dicarbonsäure und einem mindestens trifunktionellen Alkohol. Die Erfindung betrifft ferner den genannten hyperverzweigten Polyester, ein Verfahren zu seiner Herstellung und seine Verwendung zur Solubilisierung eines Wirkstoffs, der in Wasser bei 20 °C zu höchstens 10 g/L löslich ist, in wässrigen Lösungen. Kombinationen bevorzugter Merkmale mit anderen bevorzugten Merkmalen werden von der vorliegenden Erfindung umfasst.

In vielen Fällen ist es erforderlich, hydrophobe Wirkstoffe in Wasser zu solubilisieren, ohne den betreffenden Wirkstoff als solchen chemisch zu verändern. Dazu ist es beispielsweise möglich, eine Emulsion herzustellen, wobei sich der betreffende Wirkstoff in der Ölphase der Emulsion befindet. Bei vielen pharmazeutischen Wirkstoffen oder insbesondere Pflanzenschutzmitteln, insbesondere bei solchen, die mit einer Körperflüssigkeit bzw. in einem Pflanzensaft transportiert werden sollen, ist ein derartiges Vorgehen jedoch nicht möglich. Unter der Einwirkung von hohen Scherkräften können Emulsionen brechen. Außerdem ist eine Sterilisierung unter Erhalt der Emulsion in vielen Fällen nicht möglich.

Zusammensetzungen enthaltend einen Wirkstoff und einen hyperverzweigten Polyester sind allgemein bekannt: WO 2007/125028 offenbart ein Verfahren zur Solubilisierung von hydrophoben Wirkstoffen in wässrigem Medium, wobei man einen hyperverzweigten Polyester einsetzt, der optional mit einer Polyalkylenoxideinheit, die eine Isocyanatgruppe trägt, umgesetzt worden ist. Zur Herstellung des Polyester werden verschiedenste Dicarbonsäuren beschrieben, wie Sebacinsäure, und verschiedenste trifunktionelle Alkohole, wie Glycerin, Trimethylolpropan, Pentaerythrit und deren alkoxylierte Derivate.

Hyperverzweigte Polyester sind allgemein bekannt: WO 2009/047210 offenbart hyperverzweigte Polyester umfassend Dicarbonsäureeinheiten und trifunktionelle Alkohole, wobei als Dicarbonsäureeinheiten mit C₃-C₄₀ Alkylresten oder Alkenylresten substituierte Bernsteinsäureeinheiten beschrieben werden. Verschiedenste trifunktionelle Alkohole werden genannt, wie Glycerin, Trimethylolpropan, Pentaerythrit und deren alkoxylierten Derivate. WO 2007/068632 offenbart hyperverzweigte Polyester erhältlich durch Umsetzung von Polyisobutengruppen aufweisenden Dicarbonsäuren und trifunktionellen Alkoholen, wie Glycerin, Trimethylolpropan, Pentaerythrit und deren alkoxylierten Derivate.

Nachteilig an den bekannten hyperverzweigten Polyestern ist, dass sie nur kleine Mengen an schwerlöslichen Wirkstoffen solubilisieren können, da sie zumeist über eine gering ausgeprägte amphiphile Struktur verfügen. Außerdem sind die Polyester oft selbst nicht wasserlöslich oder nicht wasserdispergierbar, so dass sie zur Solubilisierung in wässrigen Medien nicht geeignet sind. Selbst durch eine Neutralisierung der vorhandenen Carbonsäuregruppen kann in der Regel keine Wasserlöslichkeit erzielt werden, da die Säurezahl meist sehr gering ist, z.B. unter 50 oder sogar unter 20 mg KOH pro g Polymer.

Die in WO 2007/125028 als bevorzugte Strukturen aufgeführten Polyester sind zudem hydrolytisch instabil aufgrund der relativ polaren, kurzkettigen Dicarbonsäurekomponenten (z.B. Bernsteinsäure, Adipinsäure), weshalb sie zur Herstellung von lagerstabilen, wässrigen Wirkstoffformulierungen, speziell im agrochemischen Bereich, weniger geeignet sind.

Aufgabe der vorliegenden Erfindung war es, einen alternativen hyperverzweigten Polyester zu finden, der zur Solubilisierung von schwerlöslichen Wirkstoffen geeignet ist, vor allem im wässrigen Medium. Weitere Aufgabe war ein Polyester zu finden, der möglichst hohe Mengen an Wirkstoff, insbesondere agrochemischen Wirkstoff, solubilisieren kann und der möglichst stabil, insbesondere hydrolytisch stabil sein sollte. Des Weiteren sollte der Polyester selbst wasserlöslich oder wasserdispergierbar sein, entweder durch Funktionalisierung mittels einer Polyalkylenoxidgruppe und/oder einer funktionalen C₁-C₂₄ Endgruppe enthaltend eine Säuregruppe oder zwei Alkoholgruppen, und/oder durch die Existenz zahlreicher, ggf. (teil)neutralisierbarer Carbonsäuregruppen. Schließlich war es auch Aufgabe, einen hyperverzweigten Polyester zu finden, der leicht aus kommerziell erhältlichen Chemikalien und industriell zuverlässig herstellbar ist.

Die Aufgabe wurde gelöst durch eine Zusammensetzung enthaltend einen Wirkstoff, der in Wasser bei 20 °C zu höchstens 10 g/L löslich ist, und einen hyperzverzweigten Polyester basierend auf einer hydrophoben Dicarbonsäure und einem mindestens trifunktionellen Alkohol, wobei die hydrophobe Dicarbonsäure eine aliphatische C₁₀-₃₂ Dicarbonsäure, eine Polyisobutylengruppe aufweisende Dicarbonsäure und/oder eine C₃-C₄₀ Gruppe aufweisend Bernsteinsäureeinheit ist, und der mindestens trifunktionelle Alkohol Trimethylolethan, Trimethylolpropan, Bis(trimethylolpropan), Pentaerythrit, oder ein alkoxyliertes Derivat davon oder ein alkoxyliertes Derivat von Glycerin ist, und wobei der hyperverzweigte Polyester verknüpft ist mit einer Polyalkylenoxidgruppe und/oder einer funktionalen C₁-C₂₄ Endgruppe enthaltend eine Säuregruppe oder zwei Alkoholgruppen.

In einer bevorzugten Ausführungsform ist die hydrophobe Dicarbonsäure eine aliphatische C₁₀-C₃₂ Dicarbonsäure. In einer weiteren bevorzugten Ausführungsform ist die hydrophobe Dicarbonsäure eine Polyisobutylengruppe aufweisende Dicarbonsäure. In einer weiteren bevorzugten Ausführungsform ist die hydrophobe Dicarbonsäure eine C₃-C₄₀ Gruppe aufweisende Bernsteinsäureeinheit. In einer weiteren bevorzugten Ausführungsform ist die hydrophobe Dicarbonsäure eine Polyisobutylengruppe aufweisende Dicarbonsäure und/oder eine C₃-C₄₀ Gruppe aufweisende Bemsteinsäureeinheit.

Der Wirkstoff ist in Wasser bei 20 °C zu höchstens 10 g/L, bevorzugt zu höchstens 2 g/l, besonders bevorzugt zu höchstens 0,5 g/l und speziell zu höchstens 0,1 g/l löslich. Die Zusammensetzung kann einen oder mehrere verschiedene Wirkstoffe enthalten. Beispiele für Wirkstoffe sind agrochemische Wirkstoffe, kosmetische Wirkstoffe, pharmazeutische Wirkstoffe oder Nahrungsergänzungsmittel (wie Vitamine und Carotinoide). Bevorzugte Wirkstoffe sind agrochemische Wirkstoffe.

Beispiele für kosmetische Wirkstoffe sind kosmetische Öle, Riech und Aromastoffe, Vitamine oder UV-Absorber. Kosmetische Öle sind unter anderem Erdnussöl, Jojobaöl, Kokosnussöl, Mandelöl, Olivenöl, Palmöl, Ricinusöl, Sojaöl, Weizenkeimöl oder etherische Öle wie Latschenkiefernöl, Lavendelöl, Rosmarinöl, Fichtennadelöl, Kiefemnadelöl, Eukalyptusöl, Pfefferminzöl, Salbeiöl, Bergamotteöl, Terpentinöl, Melissenöl, Wacholderöl, Zitronenöl, Anisöl, Kardamomöl, Campheröl etc. oder deren Mischungen. UV-Absorber sind unter anderem 2-Hydroxy-4-methoxybenzophenon, 2,2',4,4'-Tetrahydroxybenzbphenon, 2,2'-Dihydroxy-4,4'-dimethoxybenzophenon, 2,4-Dihydroxybenzophenon, 2-Cyano-3,3-diphenylacrylsäure-2'-ethylhexylester, 2,4,6-Trianilino-p-(carbo-2'-ethylhexyl-1'-oxi)-1,3,5-triazin, 3-(4-Methoxybenzyliden)-campher, N,N-Dimethyl-4-aminobenzoesäure-2-ethylhexylester, Salicylsäure-3,3,5-trimethylcyclohexylester, 4-Isopropyl-dibenzoylmethan, p-Methoxyzimtsäure-2-ethylhexylester und p-Methoxyzimtsäure-2-isoamylester sowie deren Mischungen.

Beispiele für Riech- und Aromastoffe sind wie in der WO 01/49817, oder in "Flavors and Fragrances", Ullmann's Encyclopedia of Industrial Chemistry, Wiley-VCH, 2002 beschrieben, auf die ausdrücklich Bezug genommen wird.

Beispiele für Vitamine sind Vitamine, Provitamine und Vitaminvorstufen aus den Gruppen A, C, E und F, insbesondere 3,4-Didehydroretinol, beta-Carotin (Provitamin des Vitamin A), Ascorbinsäure (Vitamin C), sowie die Palmitinsäureester, Glucoside oder Phosphate der Ascorbinsäure, Tocopherole, insbesondere alpha-Tocopherol sowie seine Ester, z.B. das Acetat, das Nicotinat, das Phosphat und das Succinat; weiterhin Vitamin F, worunter essentielle Fettsäuren, besonders Linolsäure, Linolensäure und Arachidonsäure, verstanden werden.

Als Beispiele für pharmazeutische Wirkstoffe seien hier genannt: Benzodiazepine, Antihypertensiva, Vitamine, Cytostatika insbesondere Taxol, Anästhetika, Neuroleptika, Antidepressiva, antiviral wirksame Mittel wie beispielsweise Anti-HIV wirksame Mittel, Antibiotika, Antimykotika, Antidementiva, Fungizide, Chemotherapeutika, Urologika, Thrombozytenaggregationshemmer, Sulfonamide, Spasmolytika, Hormone, Immunglobuline, Sera, Schilddrüsentherapeutika, Psychopharmaka, Parkinsonmittel und andere Antihyperkinetika, Ophthalmika, Neuropathiepräparate, Calciumstoffwechselregulatoren, Muskelrelaxantia, Narkosemittel, Lipidsenker, Lebertherapeutika, Koronarmittel, Kardiaka, Immuntherapeutika, regulatorische Peptide und ihre Hemmstoffe, Hypnotika, Sedativa, Gynäkologika, Gichtmittel, Fibrinolytika, Enzympräparate und Transportproteine, Enzyminhibitoren, Emetika, Durchblutungsfördernde Mittel, Diuretika, Diagnostika, Corticoide, Cholinergika, Gallenwegstherapeutika, Antiasthmatika, Broncholytika, Betarezeptorenblocker, Calciumantagonisten, ACE-Hemmer, Arteriosklerosemittel, Antiphlogistika, Antikoagulantia, Antihypotonika, Antihypoglykämika, Antihypertonika, Antifibrinolytika, Antiepileptika, Antiemetika, Antidota, Antidiabetika, Antiarrhythmika, Antianämika, Antiallergika, Anthelmintika, Analgetika, Analeptika, Aldosteronantagonisten, Abmagerungsmittel.

Der Begriff agrochemische Wirkstoffe (im folgenden auch Pestizide genannt) bezeichnet mindestens einen Wirkstoff ausgewählt aus der Gruppe der Fungizide, Insektizide, Nematizide, Herbizide, Safener und/oder Wachstumsregulatoren. Bevorzugte Pestizide sind Fungizide, Insektizide und Herbizide, insbesondere Insektizide. Auch Mischungen von Pestiziden aus zwei oder mehr der vorgenannten Klassen können verwendet werden. Der Fachmann ist vertraut mit solchen Pestiziden, die beispielsweise in Pesticide Manual, 14th Ed. (2006), The British Crop Protection Council, London, gefunden werden können. Geeignete Insektizide sind Insektizide der Klasse der Carbamate, Organophophate, Organochlor-Insektizide, Phenylpyrazole, Pyrethroide, Neonicotinoide, Spinosine, Avermectine, Milbemycine, Juvenil Hormon Analoga, Alkylhalide, Organozinn-Verbindungen, Nereistoxin-Analoga, Benzoylharnstoffe, Diacylhydrazine, METI Akarizide, sowie Insektizide wie Chloropicrin, Pymetrozin, Flonicamid, Clofentezin, Hexythiazox, Etoxazol, Diafenthiuron, Propargit, Tetradifon, Chlorfenapyr, DNOC, Buprofezin, Cyromazin, Amitraz, Hydramethylnon, Acequinocyl, Fluacrypyrim, Rotenon, oder deren Derivate. Geeignete Fungizide sind Fungizide der Klassen Dinitroaniline, Allylamine, Anilinopyrimidine, Antibiotica, aromatische Kohlenwasserstoffe, Benzolsulfonamide, Benzimidazole, Benzisothiazole, Benzophenone, Benzothiadiazole, Benzotriazine, Benzylcarbamate, Carbamate, Carboxamide, Carbonsäureamide, Chloronitrile, Cyanoacetamidoxime, Cyanoimidazole, Cyclopropancarboxamide, Dicarboximide, Dihydrodioxazine, Dinitrophenylcrotonate, Dithiocarbamate, Dithiolane, Ethylphosphonate, Ethylaminothiazolcarboxamide, Guanidine, Hydroxy-(2-amino-)pyrimidine, Hydroxyanilide, Imidazole, Imidazolinone, Anorganika, Isobenzofuranone, Methoxyacrylate, Methoxycarbamate, Morpholine, N-Phenylcarbamate, Oxazolidinedione, Oximinoacetate, Oximinoacetamide, Peptidylpyrimidinnucleoside, Phenylacetamide, Phenylamide, Phenylpyrrole, Phenylhamstoffe, Phosphonate, Phosphorothiolate, Phthalamsäuren, Phthalimide, Piperazine, Piperidine, Propionamide, Pyridazinone, Pyridine, Pyridinylmethylbenzamide, Pyrimidinamine, Pyrimidine, Pyrimidinonhydrazone, Pyrroloquinolinone, Quinazolinone, Chinoline, Chinone, Sulfamide, Sulfamoyltriazole, Thiazolecarboxamide, Thiocarbamate, Thiophanate, Thiophencarboxamide, Toluamide, Triphenylzinn Verbindungen, Triazine, Triazole. Geeignete Herbizide sind Herbizide der Klassen der Acetamide, Amide, Aryloxyphenoxypropionate, Benzamide, Benzofuran, Benzoesäuren, Benzothiadiazinone, Bipyridylium, Carbamate, Chloroacetamide, Chlorcarbonsäuren, Cyclohexanedione, Dinitroaniline, Dinitrophenol, Diphenylether, Glycine, Imidazolinone, Isoxazole, Isoxazolidinone, Nitrile, N-Phenylphthalimide, Oxadiazole, Oxazolidindione, Oxyacetamide, Phenoxycarbonsäuren, Phenylcarbamate, Phenylpyrazole, Phenylpyrazoline, Phenylpyridazine, Phosphinsäuren, Phosphoroamidate, Phosphorodithioate, Phthalamate, Pyrazole, Pyridazinone, Pyridine, Pyridincarbonsäuren, Pyridinecarboxamide, Pyrimidindione, Pyrimidinyl(thio)benzoate, Chinolincarbonsäuren, Semicarbazone, Sulfonylaminocarbonyltriazolinone, Sulfonylharnstoffe, Tetrazolinone, Thiadiazole, Thiocarbamate, Triazine, Triazinone, Triazole, Triazolinone, Triazolinone, Triazolocarboxamide, Triazolopyrimidine, Triketone, Uracile, Harnstoffe.

In einer Ausführungsform enthält das Pestizid ein Insektizid, bevorzugt besteht das Pestizid aus mindestens einem Insektizid. Bevorzugte Insektizide sind Fipronil, Allethrin, Alpha-Cypermethrin, Beta-Cyfluthrin, Bifenthrin, Bioallethrin, 4-Chlor-2-(2-chlor-2methylpropyl)-5-[(6-iod-3-pyridinyl)methoxy]-3(2H)-pyridazinone (CAS-RN: 120955-77-3), Chlorfenapyr, Chlorpyrifos, Cyfluthrin, Cyhalothrin, Cypermethrin, Deltamethrin, Etofenprox, Fenoxycarb, Flufenoxuron, Hydramethylnon, Metaflumizone, Permethrin, Pyriproxifen, Silafluofen, Tebufenozide und Tralomethrin. Besonders bevorzugte Insektizide sind Fipronil, Alpha-Cypermethrin, Bifenthrin, Chlorfenapyr, Cyfluthrin, Cypermethrin, Deltamethrin, Etofenprox, Hydramethylnon, Metaflumizone, Permethrin. Ganz besonders bevorzugte Insektizide sind Fipronil, Alpha-Cypermethrin, Deltamethrin, Chlorfe-napyr, Hydramethylnon und Metaflumizone. Insbesondere bevorzugtes Insektizid ist Fipronil. In einer weiteren Ausführungsform enthält das Pestizid ein Fungizid, bevorzugt besteht das Pestizid aus mindestens einem Fungizid. Bevorzugte Fungizide sind Pyraclostrobin, Metconazol und Epoxiconazol. In einer weiteren Ausführungsform enthält das Pestizid ein Herbizid, bevorzugt besteht das Pestizid aus mindestens einem Herbizid. In einer weiteren Ausführungsform enthält das Pestizid einen Wachstumsregulator, bevorzugt besteht das Pestizid aus mindestens einem Wachstumsregulator.

Die erfindungsgemäße Zusammensetzung umfasst üblicherweise 0.1 bis 70 Gew.% Wirkstoff, bevorzugt 1 bis 60 Gew.%, insbesondere 3 bis 50 Gew.%, bezogen auf die Zusammensetzung. Das Gewichtsverhältnis von dem hyperverzweigten Polyester zu dem Wirkstoff liegt meist im Bereich 1 : 2 bis 25 : 1.

Dendrimere und hyperverzweigte Polymere sind Bezeichnungen für Polymere, die sich durch eine stark verzweigte Struktur und eine hohe Funktionalität auszeichnen. Zwischen Dendrimeren und hyperverzweigten Polymeren bestehen aber dennoch deutliche Unterschiede im Aufbau: Dendrimere sind molekular einheitliche Makromoleküle mit einem hoch symmetrischen Aufbau. Dendrimere lassen sich, ausgehend von einem zentralen Molekül, durch kontrollierte, schrittweise Verknüpfung von jeweils zwei oder mehr di- oder mehrfach funktionellen Monomeren mit jedem bereits gebundenen Monomer herstellen. Dabei vervielfacht sich mit jedem Verknüpfungsschritt die Zahl der Monomerendgruppen (und damit der Verknüpfungen) um den Faktor 2 oder höher, und man erhält generationenweise aufgebaute, monodisperse Polymere mit baumartigen Strukturen, im Idealfall kugelförmig, deren Äste jeweils exakt dieselbe Anzahl von Monomereinheiten enthalten. Aufgrund der verzweigten Struktur sind die Polymereigenschaften vorteilhaft, beispielsweise beobachtet man eine überraschend geringe Viskosität und eine hohe Reaktivität aufgrund der hohen Anzahl funktioneller Gruppen an der Kugeloberfläche. Allerdings wird die Herstellung der monodispersen Dendrimere dadurch verkompliziert, dass bei jedem Verknüpfungsschritt Schutzgruppen eingeführt und wieder entfernt werden müssen und vor Beginn jeder neuen Wachstumsstufe intensive Reinigungsoperationen erforderlich sind, weshalb man Dendrimere üblicherweise nur im Labormaßstab herstellt.

Im Gegensatz dazu sind hyperverzweigte Polymere sowohl molekular wie strukturell uneinheitlich, d.h. die Moleküle des Polymers weisen sowohl eine Verteilung hinsichtlich des Molekulargewichtes wie hinsichtlich der Struktur der Moleküle auf. Sie werden durch einen nicht-generationenweisen Aufbau erhalten. Es ist daher auch nicht notwendig, Zwischenprodukte zu isolieren und zu reinigen. Hyperverzweigte Polymere können durch einfaches Vermischen der zum Aufbau erforderlichen Komponenten und deren Reaktion in einer sogenannten Ein-Topf-Reaktion erhalten werden. Hyperverzweigte Polymere können dendrimere Substrukturen aufweisen. Daneben weisen sie aber auch lineare Polymerketten und ungleiche Polymeräste auf.

Zur Synthese von hyperverzweigten Polymeren eignen sich insbesondere so genannte ABₓ-Monomere. Diese weisen zwei verschiedene funktionelle Gruppen A und B in einem Molekül auf, die unter Bildung einer Verknüpfung intermolekular miteinander reagieren können. Die funktionelle Gruppe A ist dabei nur einmal pro Molekül enthalten und die funktionelle Gruppe B zweifach oder mehrfach. Durch die Reaktion der besagten ABₓ-Monomere miteinander entstehen unvernetzte Polymere mit einer hohen Anzahl an Verzweigungstellen. Die Polymere weisen an den Kettenenden fast ausschließlich B-Gruppen auf.

Weiterhin lassen sich hyperverzweigte Polymere über die Aₓ + B_{y}- Syntheseroute herstellen. Darin stehen Aₓ und B_{y} für zwei unterschiedliche Monomere mit den funktionellen Gruppen A und B und die Indices x und y für die Anzahl der funktionellen Gruppen pro Monomer. Bei der Aₓ + B_{y}-Synthese, dargestellt hier am Beispiel einer A₂ + B₃-Synthese, setzt man ein difunktionelles Monomer A₂ mit einem trifunktionellen Monomer B₃ um. Dabei entsteht zunächst ein 1:1-Addukt aus A und B mit im Mittel einer funktionellen Gruppe A und zwei funktionellen Gruppen B, das dann ebenfalls zu einem hyperverzweigten Polymer reagieren kann. Auch die so erhaltenen hyperverzweigten Polymere weisen überwiegend B-Gruppen als Endgruppen auf.

Die erfindungsgemäß verwendeten, nicht-dendrimeren, hyperverzweigten Polymere unterscheiden sich von Dendrimeren deutlich im Verzweigungsgrad. Der Verzweigungsgrad DB (degree of branching) der betreffenden Polymere ist definiert als DB = 100 * (T + Z) / (T + Z + L), wobei T die mittlere Anzahl der terminal gebundenen Monomereinheiten, Z die mittlere Anzahl der Verzweigungen bildenden Monomereinheiten und L die mittlere Anzahl der linear gebundenen Monomereinheiten in den Makromolekülen der jeweiligen Polymere bedeuten. Zur Definition des "Degree of Branching" siehe auch H. Frey et al., Acta Polym. 1997, 48, 30. Das Merkmal "hyperverzweigt" im Zusammenhang mit den Polymeren bedeutet im Sinne der Erfindung, dass der Verzweigungsgrad DB 10 bis 95%, bevorzugt 25 bis 90% und besonders bevorzugt 30 bis 80% beträgt. Ein Dendrimer hingegen weist die maximal mögliche Anzahl an Verzweigungsstellen auf, welche nur durch einen hochsymmetrischen Aufbau erreicht werden kann. "Dendrimere" sind im Zusammenhang mit der vorliegenden Erfindung die Polymere hingegen dann, wenn ihr Verzweigungsgrad DB = 99 bis 100% beträgt.

Die Polyester weisen in bekannter Art und Weise Ester-Verknüpfungen auf. Die Polymere umfassen als Bausteine jeweils mindestens eine hydrophobe Dicarbonsäureeinheit sowie mindestens einen trifunktionellen Alkohol. Sie können darüber hinaus weitere Bausteine umfassen. Der hyperverzweigte Polyester ist meist löslich oder dispergierbar in Wasser, das heißt, dass es möglich ist eine klare (d.h. ohne Partikel, die mit dem blossen Auge erkennbar sind) wässrige Lösung oder Dispersion herzustellen.

Als hydrophobe Dicarbonsäure ist eine aliphatische C₁₀-C₃₂ Dicarbonsäure geeignet. Bevorzugt sind Sebacinsäure, α,ω-Undecan-dicarbonsäure, α,ω-Dodecan-dicarbonsäure, Tridecan-dicarbonsäure (Brassylsäure), insbesondere Sebacinsäure.

Als hydrophobe Dicarbonsäure ist auch eine Polyisobutylengruppe aufweisende Dicarbonsäure (im folgenden auch "PIB-disäure" genannt) geeignet. In diesem Zusammenhang hat eine "Polyisobutylengruppe aufweisende Dicarbonsäure" mindestens zwei Dicarbonsäuregruppen, mindestens zwei Dicarbonsäureestergruppen oder mindestens eine Dicarbonsäureanhydridgruppe (bevorzugt hat sie eine Dicarbonsäureanhydridgruppe). Solche PIB-disäuren sind erhältlich durch Umsetzung von Polyisobutylen mit einem Enophil. In einer bevorzugten Ausführungsform handelt es sich um 1:1 (mol/mol) Reaktionsprodukte einer En-Reaktion eines Polyisobutens und des Enophils. Die Herstellung der PIB-disäure erfolgt nach den dem Fachmann bekannten Verfahren und bevorzugt wie beschrieben in den deutschen Offenlegungsschriften DE-A 195 19 042, dort bevorzugt von S. 2, Z. 39 bis S. 4, Z. 2 und besonders bevorzugt von S. 3, Z. 35 bis 58, und DE-A 43 19 671, dort bevorzugt von S. 2, Z. 30 bis Z. 68, und DE-A 43 19 672, dort bevorzugt von S. 2, Z. 44 bis S. 3, Z. 19, in den dort beschriebenen Verfahren zur Umsetzung von Polyisobutylenen mit Enophilen. Bevorzugt handelt es sich bei den Polyisobutenen um solche, die zu mindestens 60 mol-% Endgruppen aus Vinyl-Isomer und / oder Vinyliden-Isomer aufweisen.

Geeignete Enophile sind Fumarsäuredichlorid, Fumarsäure, Itaconsäure, Itaconsäuredichlorid, Maleinsäuredichlorid, Maleinsäureanhydrid und/oder Maleinsäure, bevorzugt Maleinsäureanhydrid oder Maleinsäuredichlorid, besonders bevorzugt Maleinsäureanhydrid.

Das zahlenmittlere Molekulargewicht Mₙ der PIB-säure beträgt bevorzugt mindestens 100 g/mol, besonders bevorzugt mindestens 200 g/mol. In der Regel beträgt das zahlenmittlere Molgewicht Mₙ bis zu 5000, besonders bevorzugt bis zu 2000 g/mol. In einer besonders bevorzugten Ausführungsform weisen die PIB-säuren ein zahlenmittleres Molekulargewicht Mₙ von 1000 +/- 500 g/mol auf.

Die PIB-disäure weist bevorzugt eine Struktur der allgemeinen Formel (la), (Ib) oder (Ic) auf, worin PIB eine durch eine beliebige Polymerisation erhaltene Polyisobutylenylgruppe mit einem zahlenmittleren Molekulargewicht Mₙ von 100 bis 100000 Dalton sein kann. Bevorzugt ist Formel (la), also das PIB-Bernsteinsäureanhydrid.

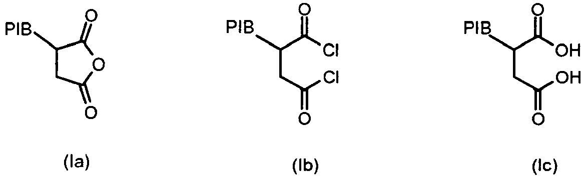

Das zahlenmittlere Molekulargewicht Mₙ des so erhältlichen und bevorzugten, mit einer Polyisobutylenylgruppe substituierten Bernsteinsäureanhydridderivats, sogenanntes "PIBSA", kann über die Verseifungszahl gem. DIN 53401 in der Einheit mg KOH/g Substanz charakterisiert werden. Die Synthese von PIBSA ist in der Literatur als En-Reaktion zwischen Maleinsäureanhydrid und Polyisobutenen bekannt (siehe z. B. DE-A 43 19 672, EP-A 156 310).

Während der En-Reaktion wird eine neue α-Olefingruppe am Kettenende erzeugt, die ihrerseits wieder reaktiv ist. Es ist dem Fachmann bekannt, dass eine Umsetzung mit weiterem Maleinsäureanhydrid ein Produkt liefert, das pro reaktivem Kettenende des Polyisobutens somit zwei Bernsteinsäureanhydrid-Gruppen tragen kann. Das bedeutet, dass ein Polyisobuten aus der BF₃-Katalyse je nach Reaktionsführung der En-Reaktion eine oder auch zwei Bernsteinsäureanhydrid-Gruppen pro Kette tragen kann. Folglich können Polyisobutene aus der lebenden kationischen Polymerisation bei der Umsetzung mit Maleinsäureanhydrid ebenfalls einfach oder zweifach pro reaktivem Kettenende substituiert sein. So sind Polyisobutene nicht nur mit einem, sondern auch mit zwei und mehr Bernsteinsäureanhydrid-Gruppen pro Molekül möglich.

Da bei der Umsetzung mit Maleinsäureanhydrid eine neue Doppelbindung entsteht, die ebenfalls mit Maleinsäureanhydrid reagieren kann, weisen die so erhältlichen, mit einer Polyisobutylengruppe substituierten Bernsteinsäureanhydride, im Allgemeinen ein Verhältnis von 0,9 bis 1,5, bevorzugt von 0,9 bis 1,1 Bernsteinsäureanhydridgruppen pro Polyisobutylenkette auf. Besonders bevorzugt trägt jede Polyisobutylenkette nur eine Bernsteinsäureanhydridgruppe.

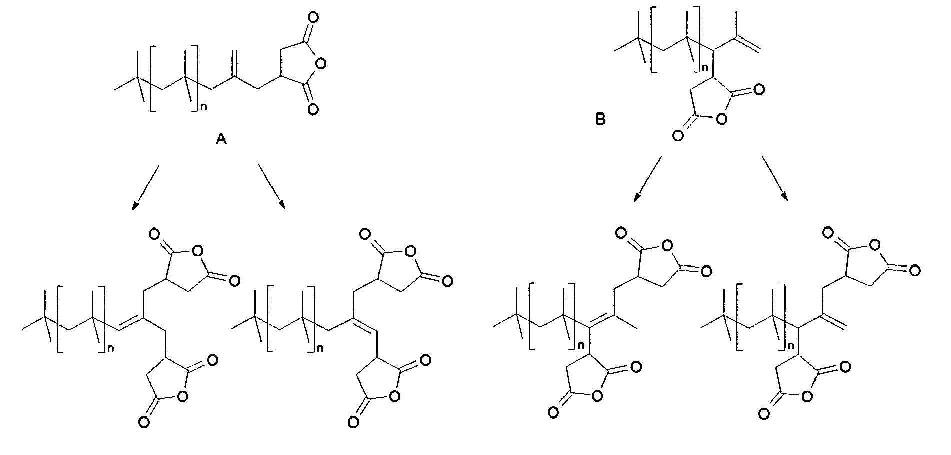

Oben ist eine beispielhafte Darstellung der Produktisomeren der einfachen En-Reaktion und zweifachen En-Reaktion eines idealen Polyisobutens mit einem einzigen reaktiven Kettenende dargestellt. Abgebildet sind Isomere mit einer (alpha-Olefin PIBSA, "A"; beta-Olefin PIBSA, "B") oder zwei Bemsteinsäureanhydrid-Gruppe(n) an einem Kettenende. Analog sind aber PIBSA mit zwei und mehr Kettenenden entsprechend mit einem oder zwei Bernsteinsäureanhydrid-Resten pro Kettenende in den verschiedenen isomeren Varianten der Mono- und Disubstitution möglich. Die Anzahl an möglichen Isomeren steigt somit mit der Zahl der Kettenenden stark an. Dem Fachmann ist bekannt, dass je nach Reaktionsführung unterschiedliche Substitutionsmuster mit unterschiedlichen Isomerengehalten der PIBSA realisiert werden können.

Der Funktionalisierungsgrad, d.h. der Anteil der mit dem Enophil umgesetzten α- oder β-olefinischen Endgruppen im Polyisobuten, der mit terminalen Bernsteinsäureanhydrid-Gruppen modifizierten Polyisobutylen-Derivate beträgt in Summe mindestens 65 mol-%, bevorzugt mindestens 75 mol-% und ganz besonders bevorzugt mindestens 85 mol-%. Bei den Polymeren mit lediglich einem reaktiven Kettenende bezieht sich der Funktionalisierungsgrad nur auf diese eine funktionale Gruppe mit den beiden möglichen Isomeren α- und β-Olefin PIBSA. Bei den zweifach und mehrfach substituierten PIBSA-Derivaten beziehen sich die Angaben der Funktionalisierungsgrade auf die Gesamtzahl aller funktionaler Gruppen innerhalb einer Molekülkette. Dabei sind, je nachdem ob Mono- oder Di-Substitution an einem Kettenende vorliegt, oben abgebildete Isomere in wechselnden Anteilen vorhanden.

Bei den nicht funktionalisierten Kettenenden kann es sich sowohl um solche handeln, die überhaupt keine reaktive Gruppe (d.h. keinen α- oder β-Olefinrest) aufweisen, wie um solche, die zwar eine reaktive Gruppe (α- oder β-Olefinrest) aufweisen, diese aber im Zuge der En-Reaktion nicht mit Maleinsäureanhydrid umgesetzt wurden. Zusammenfassend bezieht sich somit der Funktionalisierungsgrad nur auf die Anzahl aller in einer Polymerkette vorhandenen funktionalen Gruppen, nicht aber auf deren mögliche Isomere.

Daneben ist auch die Co-Polymerisation von Maleinsäureanhydrid und Polyisobutenen beschrieben, beispielsweise in WO 90/03359, EP B1 644 208, EP B1 744 413. Die so hergestellten Produkte sind unter dem Namen polyPIBSA bekannt. Im Vergleich zur En-Reaktion spielt aber die Co-Polymerisation eine eher untergeordnete Rolle. Bei dieser Co-Polymerisation von Maleinsäureanhydrid und Polyisobutenen entstehen unter Einsatz von Radikalstartern alternierende Co-Polymere mit Kammstruktur. Sowohl von Maleinsäureanhydrid als auch von Polyisobutenen mit olefinischen Endgruppen sind keine Homopolymere bekannt. Somit kann angenommen werden, dass polyPIBSAs eine streng alternierende Struktur aufweisen. Ein Funktionalisierungsgrad wie bei den PIBSAs mit endständigen Succinanhydrideinheiten aus der En-Reaktion kann nicht angegeben werden. Nachfolgend ist die Struktur von polyPIBSAs abgebildet.

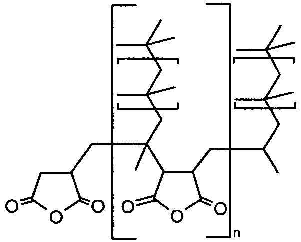

Als hydrophobe Dicarbonsäure geeignet sind auch C₃-C₄₀ Gruppen aufweisende Bernsteinsäureeinheiten, bevorzugt substituierte Bernsteinsäureeinheiten der Formel (II) 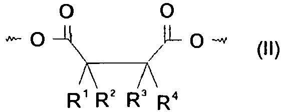 wobei R¹, R², R³ und R⁴ unabhängig voneinander für H, einen C₃ bis C₄₀-Al-kylrest oder einen C₃ bis C₄₀-Alkenylrest stehen, mit der Maßgabe, dass mindestens einer der Reste R¹, R², R³ und R⁴ nicht für H steht. Bevorzugt handelt es sich um Alkenylreste. Bevorzugt stehen zwei oder drei der Reste R¹, R², R³ oder R⁴ für H und besonders bevorzugt stehen drei der Reste für H, d.h. die Bernsteinsäureeinheit weist nur eine Alkyl- oder Alkenylgruppe auf. Der eine Substituent kann sich in Position R¹ oder R³ befinden.

Die Alkylreste können linear oder verzweigt sein. Bevorzugt handelt es sich um C₄ bis C₃₀-Reste, besonders bevorzugt C₆ bis C₂₈-Reste, ganz besonders bevorzugt C₈ bis C₂₆-Reste und beispielsweise um C₁₀ bis C₂₀-Reste. Besonders bevorzugt sind die Alkylketten linear. Beispielsweise kann es sich um Butyl-, Pentyl-, Hexyl-, Heptyl-, Octyl-, Decyl-, Dodecyl-, Tetradecyl-, Hexadecyl-, Octadecyl- oder Isooctadecylreste handeln. bevorzugt handelt es sich um Decyl-, Dodecyl-, Tetradecyl-, Hexadecyl-, Octadecyl- oder Isooctadecylreste. Falls die Reste verzweigt sind, sollte bevorzugt nicht mehr als eine Verzweigung pro 3 C-Atome des Restes vorhanden sein, besonders bevorzugt nicht mehr als eine Verzweigung pro 4 C-Atome des Restes.

Alkenylreste weisen eine oder auch mehrere Doppelbindungen auf. Bevorzugt handelt es sich um Alkenylreste mit einer Doppelbindung. Die Alkenylreste können linear oder verzweigt sein. Falls die Reste verzweigt sind, sollte bevorzugt nicht mehr als eine Verzweigung pro 3 C-Atome des Restes vorhanden sein, bevorzugt nicht mehr als eine Verzweigung pro 4 C-Atome des Restes. Bevorzugt handelt es sich um C₄ bis C₃₀-Reste, besonders bevorzugt C₆ bis C₂₈-Reste, ganz besonders bevorzugt C₈ -bis C₂₆-Reste und beispielsweise um C₁₀ bis C₂₀-Reste. Bevorzugt kann es sich bei den Alkenylresten um n- oder iso-Hexenyl-, n- oder iso-Heptenyl-, n- oder iso-Octenyl-, n- oder iso-Octadienyl-, n- oder iso-Nonenyl-, n- oder iso-Decenyl-, n- oder iso-Dodecenyl-, n- oder iso-Tetradecenyl-, n- oder iso-Hexadecenyl-, n- oder iso-Octadecenyl- oder Tetrapropenylreste handeln. Besonders bevorzugte handelt es sich bei den Alkenylresten um n- oder iso-Octenyl-, n- oder iso-Dodecenyl-, n- oder iso-Tetradecenyl-, n- oder iso-Hexadecenyl-, n- oder iso-Octadecenyl- oder Tetrapropenylreste.

Zur Synthese der hyperverzweigten Polyester kann in der beschriebenen Art und Weise substituierte Bernsteinsäure eingesetzt werden. Bevorzugt kann die Bemsteinsäure aber in Form aktivierter Derivate, insbesondere in Form von Halogeniden, Estern oder Anhydriden eingesetzt werden. Derivate sind insbesondere die betreffenden Anhydride in monomerer oder auch polymerer Form, Mono- oder Dialkylester, bevorzugt Mono- oder Di-C₁-C₄-Alkylester, besonders bevorzugt Mono- oder Dimethylester oder die entsprechenden Mono- oder Diethylester, ferner Mono- und Divinylester sowie gemischte Ester, bevorzugt gemischte Ester mit unterschiedlichen C₁-C₄-Alkylkomponenten, besonders bevorzugt gemischte Methylethylester.

Besonders bevorzugt werden Bernsteinsäureanhydride als Ausgangsmaterial eingesetzt. Neben der hohen Reaktivität der Anhydride hat der Einsatz der Anhydride den Vorteil, dass Alkenylbemsteinsäureanhydride durch Umsetzen von Maleinsäureanhydrid mit Olefinen, welche in der Allylstellung ein H-Atom aufweisen (die sogenannte En-Reaktion), besonders einfach und kostengünstig hergestellt werden können. Durch Umsetzung von linearen α-Olefinen können Alkenylbernsteinsäureanhydride mit n-AIkenylresten erhalten werden, isomerisierte Olefine mit nicht endständigen Doppelbindungen ergeben mit iso-Alkenylresten substituierte Bernsteinsäureanhydride. Als Olefine können auch reaktive Oligo- oder Polyolefine eingesetzt werden, wobei bevorzugt keine reaktiven Polyisobutene eingesetzt werden. Die Herstellung von Alkenylbernsteinsäureanhydriden (auch als ASA bekannt) mittels der En-Reaktion ist beispielsweise in WO 97/23474 oder DE 195 19 042 sowie der darin zitierten Literatur ausführlich dargestellt.

Bevorzugt eingesetzte, mit Alkenylgruppen substituierte Bernsteinsäureanhydride sind n- oder iso-Hexenylbernsteinsäureanhydrid, n- oder iso-Heptenylbernsteinsäureanhydrid, n- oder iso-Octenylbernsteinsäureanhydrid, n- oder iso-Octadienylbemsteinsäureanhydrid, n- oder iso-Nonenylbernsteinsäureanhydrid, n- oder iso-Decenylbemsteinsäureanhydrid, n- oder iso-Dodecenylbernsteinsäureanhydrid (DDSA), n- oder iso-Tetradecenylbernsteinsäureanhydrid, n- oder iso-Hexadecenylbernsteinsäureanhydrid, n- oder iso-Octadecenylbemsteinsäureanhydrid, Tetrapropenylbernsteinsäureanhydrid, 2-Dodecenyl-3-tetradecenylbensteinsäureanhydrid. Selbstverständlich können auch Gemische verschiedener substituierter Anhydride eingesetzt werden.

Besonders bevorzugte Produkte sind n- oder iso-Octenylbernsteinsäureanhydrid, n- oder iso-Dodecenylbernsteinsäureanhydrid (DDSA), n- oder iso-Tetradecenylbernsteinsäureanhydrid, n- oder iso-Hexadecenylbernsteinsäureanhydrid, n- oder iso-Octadecenylbernsteinsäureanhydrid, Tetrapropenylbernsteinsäureanhydrid oder Gemische der genannten Produkte. Ganz besonders bevorzugt sind n- oder iso-Hexadecenylbernsteinsäureanhydrid, n- oder iso-Octadecenylbernsteinsäureanhydrid, oder deren Gemische.

Die Alkenylbernsteinsäuren oder Derivate oder deren Gemische können auch in Mischung mit Alkylbernsteinsäuren oder deren Derivaten eingesetzt werden.

Zur Herstellung der hyperverzweigten Polyester werden mindestens eine hydrophobe Dicarbonsäure mit mindestens einem trifunktionellen Alkohol umgesetzt, wobei man das Verhältnis der reaktiven Gruppen im Reaktionsgemisch so wählt, dass sich ein molares Verhältnis von OH-Gruppen zu Carboxylgruppen bzw. deren Derivaten von 5:1 bis 1:5, vorzugsweise von 4:1 bis 1:4, besonders bevorzugt von 3:1 bis 1:3 und ganz besonders bevorzugt von 2:1 bis 1:2 einstellt. Wenn Mischungen aus hydrophoben aliphatischen C₁₀-C₃₂ Dicarbonsäuren und/oder Polyisobutylengruppen aufweisenden Dicarbonsäuren und/oder C₃-C₄₀ Gruppe aufweisenden Bernsteinsäureeinheiten verwendet werden, wird meist die Stöchiometrie von OH-Gruppen zu Carboxylgruppen wie oben beschrieben eingehalten.

Unter trifunktionellen Alkoholen werden Alkohole mit mindestens drei Alkoholgruppen verstanden. Als mindestens trifunktioneller Alkohol sind Trimethylolethan, Trimethylolpropan, Bis(trimethylolpropan), Pentaerythrit, oder ein alkoxyliertes (bevorzugt ethoxyliertes oder propoxyliertes) Derivat davon oder ein alkoxyliertes Derivat von Glycerin geeignet. Selbstverständlich können auch Gemische mehrerer verschiedener mindestens trifunktioneller Alkohole eingesetzt werden. Bevorzugt als mindestens trifunktionelle Alkohole sind Trimethylolpropan und Pentaerythrit. Ganz besonders bevorzugt sind Glycerin und Trimethylolpropan.

Alkoxylierte Derivate von Glycerin, Trimethylolethan, Trimethylolpropan, Bis(trimethylolpropan), Pentaerythrit können in prinzipiell bekannter Art und Weise durch Alkoxylierung der Alkohole mit Alkylenoxid, wie Ethylenoxid, Propylenoxid, Butylenoxid, und/oder Pentylenoxid, erhalten werden. Bei den gemischt alkoxylierten Polyetherolen kann es sich um Copolymere handeln, bei denen beispielweise verschiedene Alkylenoxideinheiten statistisch in der Kette verteilt sind oder es kann sich um Blockcopolymere handeln.

Bevorzugt ist das alkoxylierte Derivat von Glycerin, Trimethylolethan, Trimethylolpropan, Bis(trimethylolpropan) oder Pentaerythrit mit 1,1 bis 20 Alkylenoxideinheiten, bevorzugt Ethylenoxid- und/oder Propylenoxideinheiten, alkoxyliert. Ganz besonders bevorzugt ist das alkoxylierte Derivat von Glycerin, Trimethylolpropan oder Pentaerythrit mit 1,1 bis 20 Propylenoxideinheiten alkoxyliert.

Neben den genannten Komponenten können optional noch weitere Komponenten zur Synthese der erfindungsgemäß verwendeten hyperverzweigten Polymere eingesetzt werden. Mit solchen Komponenten lassen sich die Eigenschaften der Polymere beeinflussen und optimal für den gewünschten Zweck anpassen.

So können weitere di- oder polyfunktionelle Carbonsäuren eingesetzt werden. Beispiele weiterer Carbonsäure umfassen Malonsäure, Bernsteinsäure, Glutarsäure, Adipinsäure, 1,2-, 1,3- oder 1,4-Cyclohexandicarbonsäure (Hexahydrophthalsäuren), Phthalsäure, Isophthalsäure, Terephthalsäure bzw. Derivate davon, insbesondere deren Anhydride oder Ester. Die Menge derartiger weiterer Carbonsäuren sollte aber im Regelfalle 50 mol% bzgl. der Menge aller eingesetzten Carbonsäuren (d.h. Summe aus hydrophoben Dicarbonsäuren und weiteren di- oder polyfunktionellen Carbonsäuren) zusammen nicht überschreiten.

Weiterhin können neben den trifunktionellen Alkoholen auch noch difunktionelle aliphatische, cycloaliphatische, araliphatische oder aromatische Diole eingesetzt werden. Durch die geeignete Auswahl zweiwertiger Alkohole können die Eigenschaften der Polyester beeinflusst werden. Beispiele von geeigneten Diolen sind Ethylenglykol, 1,2-Propandiol, 1,3-Propandiol, 1,4-Butandiol, 1,5-Pentandiol, 1,6-Hexandiol, 1,8-Octandiol, 1,2-, 1,3- und 1,4-Cyclohexandiol, 1,3- und 1,4-Bis(hydroxymethyl)cyclohexan, sowie Diethylenglykol, Triethylenglykol, Dipropylenglykol, Tripropylenglykol, Polyethylenglykole HO(CH₂CH₂O)ₙ-H oder Polypropylenglykole HO(CH[CH₃]CH₂O)ₙ-H, wobei n eine ganze Zahl und n ≥ 4 ist, Polyethylen-polypropylenglykole, wobei die Abfolge der Ethylenoxid- oder Propylenoxid-Einheiten blockweise oder statistisch sein kann, oder Polytetramethylenglykole, vorzugsweise bis zu einem Molgewicht von 5000 g/mol. Die zweiwertigen Alkohole können optional noch weitere Funktionalitäten wie beispielsweise Carbonyl-, Carboxy-, Alkoxycarbonyl- oder Sulfonyl-Funktionen enthalten, wie beispielsweise Dimethylolpropionsäure oder Dimethylolbuttersäure, sowie deren C₁-C₄-Alkylester, Glycerinmonostearat oder Glycerinmonooleat. Die Menge derartiger weiterer zweiwertiger Alkohole sollte aber im Regelfalle 50 mol % bzgl. der Menge aller eingesetzten Alkohole (d.h. Summe aus trifunktionellem Alkohol und difunktionellem Diol) nicht überschreiten. Bevorzugt beträgt die Menge an zweiwertigen Alkoholen maximal 30 mol %, besonders bevorzugt maximal 20 mol %. Ganz besonders bevorzugt werden nur die trifunktionellen Alkohole eingesetzt.

Die Umsetzung aller Komponenten des hyperverzweigten Polyesters kann in Gegenwart oder Abwesenheit eines Lösemittels durchgeführt werden. Als Lösemittel geeignet sind beispielsweise Kohlenwasserstoffe wie Paraffine, Aromaten, Ether und Ketone. Vorzugsweise wird die Reaktion aber frei von Lösungsmittel durchgeführt.

Die Umsetzung erfolgt in der Regel bei erhöhten Temperaturen, beispielsweise 30 bis 250°C, insbesondere 80 bis 220°C und besonders bevorzugt 80 bis 180°C.

Das während der Polymerisation (Polykondensation) gebildete Wasser bzw. die Alkohole sollten mittels geeigneter Maßnahmen aus dem Reaktionsmedium entfernt werden. Die Umsetzung kann beispielsweise in Gegenwart eines Wasser entziehenden Mittels als Additiv erfolgen, das man zu Beginn der Reaktion zusetzt. Geeignet sind beispielsweise Molekularsiebe, insbesondere Molekularsieb 4Å, wasserfreies MgSO₄ oder wasserfreies Na₂SO₄. Weiterhin können während der Reaktion gebildetes Wasser bzw. Alkohole abdestilliert werden. Dies kann auch mittels eines geeigneten Schleppmittels unter Verwendung eines Wasserabscheiders erfolgen. Bevorzugt kann das Abdestilieren unter vermindertem Druck erfolgen, beispielsweise bei einem Druck von 1 mbar bis 500 mbar.

Man kann die Umsetzung in Abwesenheit von Katalysatoren durchführen. Vorzugsweise arbeitet man jedoch in Gegenwart von mindestens einem Katalysator. Hierbei handelt es sich bevorzugt um saure anorganische, metallorganische oder organische Katalysatoren oder um Gemische aus mehreren sauren anorganischen, metallorganischen oder organischen Katalysatoren. Es ist erfindungsgemäß auch möglich, als Katalysatoren Enzyme einzusetzen, wenn deren Einsatz auch weniger bevorzugt ist.

Als saure anorganische Katalysatoren im Sinne der vorliegenden Erfindung sind beispielsweise Schwefelsäure, Sulfate und Hydrogensulfate, wie Natriumhydrogensulfat, Phosphorsäure, Phosphonsäure, hypophosphorige Säure, Aluminiumsulfathydrat, Alaun, saures Kieselgel (pH ≤ 6, insbesondere ≤ 5) und saures Aluminiumoxid zu nennen. Weiterhin sind beispielsweise Alumiumverbindungen der allgemeinen Formel Al(OR¹)₃ und Titanate der allgemeinen Formel Ti(OR¹)₄ als saure anorganische Katalysatoren einsetzbar, wobei die Reste R¹ jeweils gleich oder verschieden sein können und unabhängig voneinander gewählt sind aus C₁-C₂₀-Alkylresten, beispielsweise Methyl, Ethyl, n-Propyl, iso-Propyl, n-Butyl, iso-Butyl, sec.-Butyl, tert.-Butyl, n-Pentyl, isoPentyl, sec.-Pentyl, neo-Pentyl, 1,2-Dimethylpropyl, iso-Amyl, n-Hexyl, iso-Hexyl, sec.-Hexyl, n-Heptyl, iso-Heptyl, n-Octyl, 2-Ethylhexyl, n-Nonyl, n-Decyl, n-Dodecyl, n-Hexadecyl oder n-Octadecyl; C₃-C₁₂-Cycloalkylresten, beispielsweise Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl und Cycloheptyl. Bevorzugt sind die Reste R¹ in Al(OR¹)₃ bzw. Ti(OR¹)₄ jeweils gleich und gewählt aus n-Butyl, Isopropyl oder 2-Ethylhexyl.

Bevorzugte saure metallorganische Katalysatoren sind beispielsweise gewählt aus Dialkylzinnoxiden R¹₂SnO oder Dialkylzinndiestem R¹₂Sn(OR²)₂ wobei R¹ wie oben stehend definiert ist und gleich oder verschieden sein kann. R² kann die gleichen Bedeutungen haben wie R¹ und zusätzlich C₆-C₁₂-Aryl sein, beispielsweise Phenyl, o-, m- oder p-Tolyl, Xylyl oder Naphthyl. R² kann jeweils gleich oder verschieden sein. Beispiele sind für zinnorganische Katalysatoren sind Zinn(II)-n-octanoat, Zinn-(II)-2-ethyl-hexanoat, Zinn-(II)-laurat, Dibutylzinnoxid, Diphenylzinnoxid, Dibutylzinndichlorid, Dibutylzinndiacetat, Dibutylzinndilaurat, Dibutylzinndimaleat oder Dioctylzinndiacetat. Denkbar sind auch Antimon-, Wismut-. oder Aluminiumorganische Katalysatoren. Besonders bevorzugte Vertreter für saure metallorganische Katalysatoren sind Dibutylzinnoxid, Diphenylzinnoxid und Dibutylzinndilaurat.

Bevorzugte saure organische Katalysatoren sind saure organische Verbindungen mit beispielsweise Phosphatgruppen, Sulfonsäuregruppen, Sulfatgruppen oder Phosphonsäuregruppen. Besonders bevorzugt sind Sulfonsäuren wie beispielsweise para-Toluolsulfonsäure. Man kann auch saure Ionentauscher als saure organische Katalysatoren einsetzen, beispielsweise Sulfonsäuregruppen-haltige Polystyrolharze, die mit etwa 2 mol-% Divinylbenzol vernetzt sind.

Man kann auch Kombinationen von zwei oder mehreren der vorgenannten Katalysatoren einsetzen. Auch ist es möglich, solche organische oder metallorganische oder auch anorganische Katalysatoren, die in Form diskreter Moleküle vorliegen, in immobilisierter Form, zum Beispiel an Kieselgel oder an Zeolithen, einzusetzen. Wünscht man saure anorganische, metallorganische oder organische Katalysatoren einzusetzen, so setzt man erfindungsgemäß 0,001 bis 10 Gew.-%, bevorzugt 0,01 bis 1 Gew.-% Katalysator ein.

Die Reaktionsdauer beträgt üblicherweise 5 Minuten bis 48 Stunden, bevorzugt 30 Minuten bis 24 Stunden und besonders bevorzugt 1 Stunde bis 10 Stunden. Das Ende der Umsetzung kann man oftmals daran erkennen, dass die Viskosität der Reaktionsmischung plötzlich schnell anzusteigen beginnt. Beim beginnenden Viskositätsanstieg kann man die Reaktion abstoppen, beispielsweise durch Abkühlen. Danach kann man an einer Probe der Mischung die Carboxylgruppenanzahl im (Prä)polymer bestimmen, beispielsweise durch Titration der Säurezahl nach DIN 53402-2.

Bei der Umsetzung der beschriebenen Monomere bilden sich Esterbindungen aus. Die erhaltenen hyperverzweigten Polyester sind im Wesentlichen unvernetzt. Im Wesentlichen unvernetzt im Rahmen dieser Erfindung bedeutet, dass ein Vernetzungsgrad von weniger als 15 Gew.-%, bevorzugt von weniger als 10 Gew.-%, bestimmt über den unlöslichen Anteil des Polymeren, vorhanden ist. Der unlösliche Anteil des Polymeren wurde bestimmt durch vierstündige Extraktion mit dem gleichen Lösungsmittel, wie es für die Gelpermeationschromatographie verwendet wird, also Tetrahydrofuran, Dimethylacetamid oder Hexafluorisopropanol, je nachdem, in welchem Lösungsmittel das Polymer besser löslich ist, in einer Soxhlet-Apparatur und nach Trocknung des Rückstandes bis zur Gewichtskonstanz Wägung des verbliebenen Rückstandes.

Beim Arbeiten ohne Lösungsmittel erhält man in der Regel unmittelbar das Endprodukt, das erforderlichenfalls durch übliche Reinigungsoperationen gereinigt werden kann. Sofern ein Lösungsmittel mitverwendet wurde, kann dieses nach der Umsetzung in üblicher Weise aus der Reaktionsmischung entfernt werden, etwa durch Vakuumdestillation.

Die Synthese zeichnet sich durch ihre große Einfachheit aus und ermöglicht die Herstellung von hyperverzweigten Polyestern in einer einfachen Ein-Topf-Reaktion. Die Isolierung oder Reinigung von Zwischenstufen oder Schutzgruppen für Zwischenstufen sind nicht erforderlich. Weitere Einzelheiten zur Herstellung von hyperverzweigten Polyestern sind beispielsweise in WO 01/46296, DE 101 63 163, DE 102 19 508, DE 102 40 817 oder WO 99/16810 dargestellt. Die Herstellung der hyperverzweigten Polyester erfolgt zumeist in einem Druckbereich von 2 mbar bis 20 bar, bevorzugt bei Normaldruck, in Reaktoren oder Reaktorkaskaden, die im Batchbetrieb, halbkontinuierlich oder kontinuierlich betrieben werden. Durch die vorgenannte Einstellung der Reaktionsbedingungen und gegebenenfalls durch die Wahl des geeigneten Lösemittels können die erfindungsgemässen Produkte nach der Herstellung ohne weitere Reinigung weiterverarbeitet werden.

Bevorzugt sind hyperverzweigte Polyester, die ein gewichtsmittleres Molekulargewicht im Bereich von etwa 500 bis 100000, besonders bevorzugt von 1000 bis 50000, aufweisen. Bei einem hyperverzweigten Polyester, der verknüpft ist mit einer Polyalkylenoxidgruppe bezieht sich das Molekulargewicht nur auf den Teil des hyperverzweigten Polyesters ohne die Polyalkylenoxidgruppe. Die Bestimmung erfolgt meist per Gelpermeationschromatographie mit einem Refraktometer als Detektor. Bevorzugt wird die Bestimmung wie in den Beispielen beschrieben durchgeführt.

Die Polydispersität der erfindungsgemäß verwendeten Polyester beträgt im Allgemeinen 1,2 bis 50, bevorzugt 1,4 bis 40, besonders bevorzugt 1,5 bis 30 und ganz besonders bevorzugt 2 bis 30. Die Angaben zur Polydispersität sowie zum zahlenmittleren und gewichtsmittleren Molekulargewicht Mₙ und M_{w} beziehen sich hier auf gelpermeationschromatographische Messungen, wobei Polymethylmethacrylat als Standard und Tetrahydrofuran, Dimethylacetamid oder Hexafluorisopropanol als Elutionsmittel verwendet wurden. Die Methode ist im Analytiker Taschenbuch Bd. 4, Seiten 433 bis 442, Berlin 1984 beschrieben.

Die Art der terminalen Gruppen kann durch das Verhältnis der eingesetzten Monomere beeinflusst werden. Sollen überwiegend OH-termierte Polymere erhalten werden, so sollten die Alkohole im Überschuss eingesetzt werden. Sollen überwiegend COOHterminierte Polymere erhalten werden, so sollten die Carbonsäuren im Überschuss eingesetzt werden.

Die Zahl freier OH-Gruppen (Hydroxylzahl) des hyperverzweigten Polyesters beträgt in der Regel 10 bis 500, bevorzugt 20 bis 450 mg KOH pro Gramm Polymer und kann z.B. durch Titration nach DIN 53240-2 bestimmt werden.

Die Zahl freier COOH-Gruppen (Säurezahl) des hyperverzweigten Polyesters beträgt in der Regel 0 bis 400, bevorzugt 25 bis 300, ganz besonders bevorzugt 50 bis 250, und insbesondere 120 bis 250 mg KOH pro Gramm Polymer und kann durch Titration nach DIN 53402 bestimmt werden.

Die erfindungsgemäß verwendeten hyperverzweigten Polyester weisen im Regelfalle mindestens 4 funktionelle Gruppen auf. Die Zahl der funktionellen Gruppen ist prinzipiell nicht nach oben beschränkt. Allerdings weisen Produkte mit einer zu hohen Anzahl von funktionellen Gruppen häufig unerwünschte Eigenschaften auf, wie beispielsweise schlechte Löslichkeit oder eine sehr hohe Viskosität. Daher weisen die erfindungsgemäß verwendeten hyperverzweigten Polymere im Regelfalle nicht mehr als 100 funktionelle Gruppen auf. Bevorzugt weisen die hyperverzweigten Polymere 6 bis 50 und besonders bevorzugt 6 bis 30 funktionelle Gruppen auf.

Der hyperverzweigte Polyester ist bevorzugt verknüpft mit einer Polyalkylenoxidgruppe und/oder einer funktionalen C₁-C₂₄ Endgruppe enthaltend eine Säuregruppe oder zwei Alkoholgruppen. Besonders bevorzugt ist der hyperverzweigte Polyester verknüpft mit einer Polyalkylenoxidgruppe. Dadurch können nichtionische, amphiphile Solubilisatoren erhalten werden, da eine (Teil)Neutralisation der Carbonsäuregruppen zur Erreichung der Wasserlöslichkeit/Wasserdispergierbarkeit nicht mehr notwendig ist. Diese nichtionischen Solubilisatoren sind besonders gut geeignet zur Herstellung langzeitstabiler Wirkstoffformulierungen.

Beispiele für eine Polyalkylenoxidgruppe sind Polyethylenglykol oder Polyethylenglykolmonoalkylether mit einer Molmasse Mn von 200 bis 10000 g/mol, bevorzugt 300-2000 g/mol. Bevorzugt handelt es sich bei dem Polyethylenglykol um ein Polyethylenglykolmono-C₁-C₁₈-alkylether, insbesondere um ein Polyethylenglykolmonomethylether.

Der hyperverzweigte Polyester ist bevorzugt mit der Polyalkylenoxidgruppe mittels eines Polyisocyanat-Linkers verknüpft.

Als Linker-reaktive Gruppe kann eine Hydroxy-Gruppe am Kettenende der Polyalkylenoxidgruppe verwendet werden. Bevorzugt werden Polyethylenglykolmonoalkylether, die genau eine Linker-reaktive Gruppe am Kettenende haben. Geeignete Polyisocyanat-Linker sind Polyisocyanate mit einer Funktionalität bezüglich der Isocyanatgruppen von wenigstens 1,5, insbesondere 1,5 bis 4,5 und speziell 1,8 bis 3,5 umfassend aliphatische, cycloaliphatische und aromatische Di- und Polyisocyanate sowie die Isocyanurate, Allophanate, Uretdione und Biurete von aliphatischen, cycloaliphatischen und aromatischen Diisocyanaten. Vorzugsweise weisen die Polyisocyanate im Mittel 1,8 bis 3,5 Isocyanatgruppen pro Molekül auf. Beispiele für geeignete Polyisocyanate sind aromatische Diisocyanate wie Toluol-2,4-diisocyanat, Toluol-2,6-diisocyanat, kommerziell erhältliche Mischungen von Toluol-2,4und -2,6-diisocyanat (TDI), n-Phenylendiisocyanat, 3,3'-Diphenyl-4,4'-biphenylendiisocyanat, 4,4'-Biphenylendiisocyanat, 4,4'-Diphenylmethandiisocyanat, 2,4'-Diphenylmethandiisocyanat, 3,3'-Dichlor-4,4'-biphenylendiisocyanat, Cumen-2,4-diisocyanat, 1,5-Naphthalindiisocyanat, p-Xylylendiisocyanat, p-Phenylendiisocyanat, 4-Methoxy-1,3-phenylendiisocyanat, 4-Chlor-1,3-phenylendiisocyanat, 4-Ethoxy-1,3-phenylendiisocyanat, 2,4-Dimethylen-1,3-phenylendiisocyanat, 5,6-Dimethyl-1,3-phenylendiisocyanat, 2,4-Diisocyanatodiphenylether, aliphatische Diisocyanate wie Ethylendiisocyanat, Ethylidendiisocyanat, Propylen-1,2-diisocyanat, 1,6-Hexamethylendiisocyanat (HDI), 1,4-Tetramethylendiisocyanat, 1,10-Decamethylendiisocyanat und cycloaliphatische Diisocyanate, wie Isophorondiisocyanat (IPDI), Cyclohexylen-1,2-diisocyanat, Cyclohexylen-1,4-diisocyanat und Bis(4,4'-Isocyanatocyclohexyl)methan. Unter den Polyisocyanaten sind solche bevorzugt, deren Isocyanatgruppen sich in ihrer Reaktivität unterscheiden, wie Toluol-2,4-diisocyanat, Toluol-2,6-diisocyanat, 4'-Diphenylmethandiisocyanat, cis- und trans-Isophorondiisocyanat, oder Mischungen dieser Verbindungen.

Die Umsetzung mit dem Polyisocyanat-Linker erfolgt in Schmelze oder in einem organischen Lösungsmittel, bevorzugt in einem aprotisch-polaren organischen Lösungsmitteln oder in Gemischen solcher Lösungsmittel. Beispiele sind Ketone (beispielsweise Aceton), Butylacetat, Tetrahydrofuran (THF), Xylol, Chlorbenzol, Dimethylsulfoxid (DMSO) oder Dimethylformamid (DMF). Bevorzugte Lösungsmittel sind Butylacetat, Xylol und Aceton. Die Umsetzung erfolgt üblicherweise bei erhöhten Temperaturen, wobei sich dieTemperatur auch nach der Siedetemperatur des gewählten Lösungsmittels richtet. Die Umsetzung des Polyisocyanat-Linkers mit der ersten Komponente kann bei 20 bis 80 °C, gewünschtenfalls aber auch bis 100 °C erfolgen. Die Umsetzung der weiteren Isocyanatgruppe kann bei Temperaturen von 50 bis 100 °C erfolgen. Das Lösungsmittel kann anschliessend durch Destillation entfernt werden.

Die Umsetzung kann äquimolar erfolgen, was bedeutet, dass das Mengenverhältnis so gewählt wird, dass pro Mol umzusetzender Hydroxyl-Gruppen des Funktionalisierungsreagenz oder des linearen Polyalkylenoxids 1 Mol Diisocyanat eingesetzt wird. Bevorzugt wird mit leichtem (z.B. 0 bis 15 mol%) Überschuss der Hydroxyl-Gruppen gearbeitet, um die Menge an nicht umgesetztem Diisocyanat zu verringern. Im Falle symmetrischer Diisocyanate (wie HDI) kann es sich auch empfehlen, einen Überschuss an Diisocyanat einzusetzen und den Überschuss anschliessend destillativ zu entfernen.

Vorzugsweise wird die Umsetzung in Gegenwart eines Katalysators durchgeführt. Als Katalysatoren eignen sich beispielsweise tertiäre Amine, z. B. Triethylamin, Tri-n-propylamin, N-Methylpyrrolidin, N-Methylpiperidin und Diazabicyclooctan (DABCO), Zink-Carboxylate, Wismut-Carboxylate, Titan-Alkoholate, zinnorganische Verbindungen, insbesondere Dialkylzinn(IV)salze von aliphatischen Carbonsäuren wie Dibutylzinndilaurat und Dibutylzinndioctoat, Zinn(II)dialkanoate wie Zinndioctoat, sowie Cäsiumsalze wie Cäsiumacetat. In einer Ausführungsform sind Zink-Carboxylate, Wismut-Carboxylate, Titan-Alkoholate besonders geeignet, wobei die Carboxylate bevorzugt C₁-C₂₀ Carboxylate (wie Formiat, Acetat, Propionat, Hexanoat, Octanoat oder Neodecanoat) sind. Der Katalysator kann in Mengen von 50 bis 50000 ppm, vorzugsweise 100 bis 5000 ppm, bezogen auf gesamten Feststoff, eingesetzt werden.

Üblicherweise wird man die Umsetzung so durchführen, dass zunächst die Komponente, die Isocyanatgruppen-funktionalisiert werden soll (beispielsweise das Polyalkylenoxid), mit dem Diisocyanat in Gegenwart des Katalysators und eines Lösungsmittels solange umgesetzt wird, bis der Isocyanat-Wert im Reaktionsgemisch auf die Hälfte gesunken ist. Bei Verwendung eines leichten Hydroxygruppen-Überschusses wird solange umgesetzt, bis der theoretische Endwert dem vollständigen Umsatz der Hydroxyl-Gruppen entspricht. Dies lässt sich auf bekannte Weise beispielsweise titrimetrisch ermitteln. Danach erfolgt dann die Zugabe des hyperverzweigten Polyesters. Das molare Verhältnis von hyperverzweigtem Polyester zum Polyalkylenoxid bzw. zur funktionalen C₁-C₂₄ Endgruppe enthaltend eine Säuregruppe oder zwei Alkoholgruppen liegt dabei bei 1 : 1 bis 1 : 25, bevorzugt bei 1 : 2 bis 1 : 15. Die Reaktion wird fortgeführt, bis der Isocyanatwert auf Null gesunken ist.

Geeignete funktionale C₁-C₂₄ Endgruppen enthaltend eine Säuregruppe oder zwei Alkoholgruppen sind bevorzugt aliphatische Verbindungen mit 1 - 24 Kohlenstoffatomen, die mindestens eine Säuregruppe oder mindestens zwei Alkoholgruppen tragen. Säuregruppen sind beispielsweise eine Carbonsäuregruppe, eine Sulfonsäuregruppe, eine Sulfensäuregruppe, eine Sulfinsäuregruppe, eine Schwefelsäureestergruppe (d.h. ein organisches Sulfat), eine Phosphonsäuregruppe, eine Aminogruppe oder mindestens zwei Hydroxy-C₂-C₁₀-alkylgruppen, besonders bevorzugt eine Carbonsäuregruppe. Optional kann die funktionale C₁-C₂₄ Einheit auch mehrere der aufgeführten Gruppen zugleich enthalten. Bevorzugt basiert die funktionale C₁-C₂₄ Endgruppe auf einem zyklischen Carbonsäureanhydrid, insbesondere handelt es sich um Bernsteinsäureanhydrid. Die Säuregruppen der funktionalen C₁-C₂₄ Endgruppe können teilweise oder vollständig neutralisiert sein, bevorzugt mit Alkalimetallhydroxiden oder organischen Aminen.

In einer Ausführungsform enthält die funktionale C₁-C₂₄-Einheit zusätzlich eine Verknüpfungsgruppe, mit der die funktionalen C₁-C₂₄-Einheiten an den hyperverzweigten Polyester direkt oder mittels eines Polyisocyanat-Linkers kovalent verknüpft werden können. Geeignete Verknüpfungsgruppen können mit den OH- und/oder Carbonsäuregruppen des Polyesters reagieren. Beispiele sind Carbonsäuren, Carbonsäureester, Carbonsäureanhydride, Isocyanate, Amine und Alkohole. Weitere geeignete Verküpfungsgruppen können mit dem Polyisocyanat-Linker reagieren. Beispiele sind Alkohole oder Amine, bevorzugt Alkohole.

Der hyperverzweigte Polyester, der verknüpft ist mit funktionalen C₁-C₂₄ Endgruppen, ist üblicherweise erhältlich, bevorzugt wird es erhalten, durch Umsetzung des hyperverzweigten Polyesters mit einem Funktionalisierungsreagenz, das die funktionale C₁-C₂₄-Einheit enthaltend eine Säuregruppe, eine Aminogruppe oder mindestens zwei Hydroxygruppen und die Verküpfungsgruppe umfasst, und optional mit einem Polyisocyanat-Linker.

Geeignete Funktionalisierungsreagenzien zur direkten kovalenten Verknüpfung ohne Linker sind Anhydride. Besonders geeignet sind zyklische Carbonsäureanhydride, wie Bernsteinsäureanhydrid oder Phthalsäureanhydrid, insbesondere Bernsteinsäureanhydrid. Typischerweise werden die Anhydride mit dem hyperverzweigten Polyester unter erhöhten Temperaturen, meist bei 80 bis 200 °C, umgesetzt. Die Umsetzung kann mit oder ohne Zusatz von Lösungsmitteln erfolgen. Eine weitere Aufreinigung ist normalerweise nicht nötig.

Geeignete Funktionalisierungsreagenzien zur kovalenten Verknüpfung mittels Polyisocyanat-Linker sind Hydroxycarbonsäuren, Aminocarbonsäuren, Hydroxysulfonsäuren, Hydroxysulfate, Aminosulfonsäuren oder Aminosulfate, Hydroxyamine (wie Diethanolamin), Polyamine (z.B. Diethylentetramin), oder Polyole (z.B. Glycerin, Trimethylolpropan, Pentaerythrit). Bevorzugte Polyisocyanat-Linker hierfür sind Diisocyanate, besonders bevorzugt aliphatische Diisocyanate (wie Hexamethylendiisocyanat und Isophorondiisocyanat).

Die erfindungsgemäße Zusammensetzung ist erhältlich indem man den hyperverzweigten Polyester und den Wirkstoff, der in Wasser bei 20 °C zu höchstens 10 g/L löslich ist, in Kontakt bringt. Die Komponenten können durch allgemein bekannte Methoden, wie Mischen, Emulgieren oder Suspendieren, in Kontakt gebracht werden.

Das Gewichtsverhältnis von hyperverzweigtem Polyester zu Wirkstoff liegt meist im Bereich von 1 : 50 bis 100 : 1, bevorzugt 1 : 5 bis 50 : 1, besonders bevorzugt 1 : 2 bis 25 : 1. Der Wirkstoff kann in gelöster Form oder in fester, partikulärer Form vorliegen. Die Wirkstoffpartikel können kristallin oder amorph sein. Die Partikelgröße kann 1 nm bis 10 µm betragen.

Die Zusammensetzung kann als Feststoff, Lösung, Emulsion, Suspension oder Suspoemulsion des Wirkstoffs vorliegen. Die erfindungsgemäße Zusammensetzung ist bevorzugt eine wässrige Zusammensetzung. In einer weiteren bevorzugten Ausführungsform ist die erfindungsgemäße Zusammensetzung ein Fesstoff, wobei es sich besonders bevorzugt um eine feste Lösung (sog. Solid Solution) handelt. Bei festen Lösungen liegt der der Wirkstoff üblicherweise in amorpher Form dispergiert in einer Polymermatrix vor.

Bevorzugt enthält sie mindestens 40 Gew.%, besonders bevorzugt mindestens 60 Gew.%, und insbesondere mindestens 80 Gew.% Wasser. Üblicherweise enthält die Zusammensetzung höchstens 99 Gew.% Wasser.

Die erfindungsgemäße Zusammensetzung kann Formulierungshilfsmittel enthalten, wobei sich die Wahl der Hilfsmittel üblicherweise nach der konkreten Anwendungsform bzw. dem Wirkstoff richtet. Beispiele für geeignete Formulierungshilfsmittel sind Lösungsmittel, feste Trägerstoffe, oberflächenaktive Stoffe (wie Tenside, Schutzkolloide, Netzmittel und Haftmittel), organische und anorganische Verdicker, Bakterizide, Frostschutzmittel, Entschäumer, ggf. Farbstoffe und Kleber (z. B. für Saatgutbehandlung).

Als oberflächenaktive Stoffe (Adjuvantien, Netz-, Haft-, Dispergier- oder Emulgiermittel) kommen die Alkali-, Erdalkali-, Ammoniumsalze von aromatischen Sulfonsäuren, z. B. von Lignin- (Borresperse®-Typen, Borregaard, Norwegen), Phenol-, Naphthalin-(Morwet®-Typen, Akzo Nobel, USA) und Dibutylnaphthalinsulfonsäure (Nekal®-Typen, BASF, Deutschland), sowie von Fettsäuren, Alkyl- und Alkylarylsulfonaten, Alkyl-, Laurylether- und Fettalkoholsulfaten, sowie Salze sulfatierter Hexa-, Hepta- und Octadecanole sowie von Fettalkoholglykolethern, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen sowie Proteine, denaturierte Proteine, Polysaccharide (z.B. Methylcellulose), hydrophob modifizierte Stärken, Polyvinylalkohol (Mowiol®-Typen, Clariant, Schweiz), Polycarboxylate (Sokalan®-Typen, BASF, Deutschland), Polyalkoxylate, Polyvinylamin (Lupamin®-Typen, BASF, Deutschland), Polyethylenimin (Lupasol®-Typen, BASF, Deutschland), Polyvinylpyrrolidon und deren Copolymere in Betracht.

In einer bevorzugten Ausführungsform ist der Wirkstoff ein Pestizid und die erfindungsgemäßen Zusammensetzungen liegen in Form einer agrochemischen Formulierung vor. Geeignete agrochemische Formulierungen sind wasserlösliche Konzentrate (SL, LS), redispergierbare Konzentrate (DC), emulgierbare Konzentrate (EC), Emulsionen (EW, EO, ES, ME), Suspensionen (SC, OD, FS) oder Suspoemulsionen (SE). Bevorzugt liegt die Zusammensetzung vor in Form eines emulgierbaren Konzentrates (EC), eines Suspensionskonzentrats (SC), eines wasserlöslichen Konzentrats (SL), einer Lösung für Saatgutbehandlung (LS), oder eines redispergierbaren Konzentrats (DC).

Die agrochemischer Formulierung wird meist vor der Anwendung verdünnt, um den sogenannten Tankmix herzustellen. Zur Verdünnung kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Isophoron, stark polare Lösungsmittel, z.B. Dimethylsulfoxid, N-Methylpyrrolidon oder Wasser in Betracht. Bevorzugt wird Wasser verwendet. Es ist auch möglich, das Amphiphil erst dem Tankmix zuzusetzen. In dieser Ausführungsform liegt die erfindungsgemäße Zusammensetzung in Form eines Tankmixes vor.

Die verdünnte Zusammensetzung wird üblicherweise durch Versprühen oder Vernebeln angewendet. Zu dem Tankmix können Öle verschiedenen Typs, Netzmittel, Adjuvants, Herbizide, Bakterizide, Fungizide unmittelbar vor der Anwendung (Tankmix) zugesetzt werden. Diese Mittel können zu den erfindungsgemäßen Zusammensetzungen im Gewichtsverhältnis 1:100 bis 100:1, bevorzugt 1:10 bis 10:1 zugemischt werden. Die Pestizidkonzentration im Tankmix kann in größeren Bereichen variiert werden. Im Allgemeinen liegen sie zwischen 0,0001 und 10%, vorzugsweise zwischen 0,01 und 1 %. Die Aufwandmengen liegen bei der Anwendung im Pflanzenschutz je nach Art des gewünschten Effektes zwischen 0,01 und 2,0 kg Wirkstoff pro ha.

Die Verwendung der agrochemischen Formulierungen ist möglich zur Bekämpfung von phytopathogenen Pilzen und/oder unerwünschtem Pflanzenwuchs und/oder unerwünschtem Insekten- oder Milbenbefall und/oder zur Regulation des Wachstums von Pflanzen, wobei man die Zusammensetzung auf die jeweiligen Schädlinge, deren Lebensraum oder die vor dem jeweiligen Schädling zu schützenden Pflanzen, den Boden und/oder auf unerwünschte Pflanzen und/oder die Nutzpflanzen und/oder deren Lebensraum einwirken lässt. Weiterhin ist die Verwendung der agrochemischen Formulierungen möglich zur Bekämpfung von unerwünschtem Insekten- oder Milbenbefall auf Pflanzen und/oder zur Bekämpfung von phytopathogenen Pilzen und/oder zur Bekämpfung unerwünschten Pflanzenwuchs, wobei man Saatgüter von Nutzpflanzen mit der Zusammensetzung behandelt.

Die Erfindung betrifft auch einen hyperzverzweigten Polyester basierend auf einer hydrophoben Dicarbonsäure und einem trifunktionellen Alkohol, wobei die hydrophobe Dicarbonsäure eine aliphatische C₁₀-C₃₂ Dicarbonsäure, eine Polyisobutylengruppe aufweisende Dicarbonsäure und/oder eine C₃-C₄₀ Gruppe aufweisende Bemsteinsäureeinheit ist, und der trifunktionelle Alkohol Glycerin, Trimethylolethan, Trimethylolpropan, Bis(trimethylolpropan), Pentaerythrit, oder ein ethoxyliertes oder propoxyliertes Derivat davon ist, und wobei der hyperverzweigte Polyester verknüpft ist mit einer Polyalkylenoxidgruppe und/oder einer funktionalen C₁-C₂₄ Endgruppe enthaltend eine Säuregruppe oder zwei Alkoholgruppen. Geeignete und bevorzugte Ausführungsformen des hyperverzweigten Polyester sind wie oben beschrieben.

Die Erfindung betrifft weiterhin ein Verfahren zur Herstellung des erfindungsgemäßen hyperverzweigten Polyesters, indem die hydrophobe Dicarbonsäure und der trifunktionelle Alkohol polykondensiert werden, und anschließend mit einer Polyalkylenoxidgruppe und/oder einer funktionalen C₁-C₂₄ Endgruppe enthaltend eine Säuregruppe oder zwei Alkoholgruppen verknüpft werden. Optional können die Säuregruppen der funktionalen C₁-C₂₄ Endgruppe teilweise oder vollständig neutralisiert werden, bevorzugt mit Alkalimetallhydroxiden oder organischen Aminen.

Die Erfindung betrifft weiterhin die Verwendung des erfindungsgemäßen hyperverzweigten Polyester zur Solubilisierung eines Wirkstoffs, der in Wasser bei 20 °C zu höchstens 10 g/L löslich ist, in wässrigen Lösungen. Solubilisierung bedeutet, dass in Anwesenheit des hyperverzweigten Polyester mehr Wirkstoff in Lösung gebracht werden kann als in dessen Abwesenheit bei ansonsten gleichen Bedingungen. Bevorzugt kann mindestens die doppelte Menge, besonders bevorzuft mindestens die fünfache und insbesondere die zehnfache Menge in Lösung gebracht werden.

Vorteile der vorliegenden Erfindung sind, dass eine hohe Konzentration von Wirkstoff in Lösung gebracht werden kann; dass die Herstellung des hyperverzweigten Polyesters sehr einfach und großtechnisch möglich ist; und dass das Amphiphil selbst wasserlöslich oder wasserdispergierbar ist, z.B. nach Funktionalisierung mittels einer Polyalkylenoxidgruppe und/oder einer funktionalen C₁-C₂₄ Endgruppe enthaltend eine Säuregruppe oder zwei Alkoholgruppen, oder durch die Existenz zahlreicher, (teil)neutralisierbarer Carbonsäuregruppen. Ein weiterer Vorteil der Erfindung besteht darin, dass die hyperverzweigten Polyester besonders hydrolysestabil sind durch Verwendung der hydrophoben Dicarbonsäure und/oder durch Verwendung von stabilen Linkermolekülen, welche beispielsweise Urethanbindungen ausbilden. Hierdurch lassen sich die erfindungsgemäßen Polyester besonders gut zur Herstellung lagerstabiler Agroformulierungen einsetzen. Weitere Vorteile sind, dass die Bioverfügbartkeit der Wirkstoffe erhöht wird, dass der systemische Effekt der agrochemischen Wirkstoffe bei foliarer Aufnahme erhöht wird, dass sogar schwerlösliche agrochemische Wirkstoffe nun gelöst formuliert werden können, beispielsweise als SL (wasserlösliches Konzentrat) oder LS (Lösung für Saatgutbehandlung), dass die Verteilung der agrochemischen Wirkstoffe in der Sprühlösung verbessert ist, und dass die Mehrwegverpackungen der Wirkstoffe und die Applikationsgeräte (z.B. die Sprühgeräte für Pestizide) besser mit Wasser gereinigt werden können.

Nachfolgende Beispiele erläutern die Erfindung.

### Beispiele

Die allgemeine Bezeichnung "TMP x n EO/PO" (z.B. "TMP x 1,1 PO" oder "TMP x 12,2 EO") beschreibt ein Produkt, das je mol Trimethylolpropan mit durchschnittlich n mol (z.B. 1,1 mol) Propylenoxid (PO) oder Ethylenoxid (EO) umgesetzt worden ist.

PIBSA 1000 beschreibt ein Polyisobutylen (M = 1000 g/mol) mit terminaler Bernsteinsäureanhydrid-Gruppe. Unter Pentasize 8 bzw. Pentasize 68 werden kommerziell erhältliche Alkenylbernsteinsäureanhydride (Trigon Chemie) verstanden, wobei es sich bei den Alkenylresten um ungesättigte C18 Einheiten (Pentasize 8) bzw. um ein Gemisch aus C16/C18 Einheiten (Pentasize 68) handelt. DBTL ist die Abkürzung für den Katalysator Di-n-butylzinndilaurat

Die hyperverzweigten Polymere wurden per Gelpermeationschromatographie mit einem Refraktometer als Detektor analysiert. Als mobile Phase wurde THF verwendet, als Standard zur Bestimmung des Molekulargewichts wurde Polymethylmethacrylat (PMMA) eingesetzt. Die Säurezahl wurde jeweils nach DIN 53402 bestimmt. Die Bestimmung der OH-Zahl (mg KOH/g) erfolgte in Anlehnung an DIN 53240, Teil 2.

Die Molmassen der mit einer Polyalkylenoxidgruppe funktionalisierten Polyester wurden rechnerisch aus dem zahlenmittleren Molekulargewicht des zugrundeliegenden hyperverzweigten Kerns, dessen OH-Zahl und dem gewählten Funktionalisierungsgrad (stöchiometrisches Verhältnis von NCO-Gruppen der funkt. linearen Polymere zu den verfügbaren Hydroxyl-Gruppen des Kemmoleküls) bestimmt.

### Synthesebeispiel 1: Hyperverzweigter Polyester A.1

In einem Vierhalskolben, der mit Rührer, Innenthermometer, Stickstoffeinleitungsrohr und absteigendem Kühler mit Auffanggefäß versehen war, wurden unter Begasung mit trockenem Stickstoff 120,1 g Trimethylolpropan und 380,1 g Sebacinsäure vorgelegt und das Reaktionsgemisch unter Rühren auf 160-170 °C erhitzt. Nach einer Reaktionszeit von 1,5 h und einer abgeschiedenen Wassermenge von 32 mL (Umsatz 47%) wurde die Reaktion durch Abkühlen auf Raumtemperatur beendet. Das Polymer A.1 (Mn = 980 g/mol; Mw = 22230 g/mol; OH-Zahl: 79 mg KOH/g Polymer; Säurezahl: 182 mg KOH/g Polymer) wurde in Form einer gelb gefärbten, hoch viskosen Flüssigkeit erhalten, welche nach Neutralisierung von 75% der terminalen Carbonsäuregruppen mit Triethanolamin sehr gut wasserlöslich war. Das Polymer A.1 wurde in dieser teilneutralisierten Form für die weiteren Versuche eingesetzt.

### Synthesebeispiel 2: Hyperverzweigter Polyester A.2

In einem Vierhalskolben, der mit Rührer, Innenthermometer, Stickstoffeinleitungsrohr und absteigendem Kühler mit Auffanggefäß versehen war, wurden unter Begasung mit trockenem Stickstoff 194,6 g des trifunktionellen Alkohols TMP x 3,2 EO und 305,5 g Sebacinsäure vorgelegt, mit 0,2 g DBTL versetzt und das Reaktionsgemisch unter Rühren auf 160-170 °C erhitzt. Nach einer Reaktionszeit von 2 h und einer abgeschiedenen Wassermenge von 26 mL (Umsatz 47%) wurde die Reaktion durch Abkühlen auf Raumtemperatur beendet. Das Polymer A.2 (Mn = 960 g/mol; Mw = 9520 g/mol; OH-Zahl: 48 mg KOH/g Polymer; Säurezahl: 156 mg KOH/g Polymer) wurde in Form einer gelb gefärbten, hoch viskosen Flüssigkeit erhalten, welche nach Neutralisierung von 100% der terminalen Carbonsäuregruppen mit Triethanolamin sehr gut wasserlöslich war. Das Polymer A.2 wurde in dieser vollständig neutralisierten Form für die weiteren Versuche eingesetzt.

### Synthesebeispiel 3: Hyperverzweigter Polyester A.3

In einem Vierhalskolben, der mit Rührer, Innenthermometer, Stickstoffeinleitungsrohr und absteigendem Kühler mit Auffanggefäß versehen war, wurden unter Begasung mit trockenem Stickstoff 160,4 g des trifunktionellen Alkohols TMP x 1,1 PO und 340,2 g Sebacinsäure vorgelegt, mit 0,2 g DBTL versetzt und das Reaktionsgemisch unter Rühren auf 160°C erhitzt. Nach 4 h und einer abgeschiedenen Wassermenge von 24 mL (Umsatz 39%) wurde die Reaktion durch Abkühlen auf Raumtemperatur beendet. Das Polymer A.3 (Mn = 820 g/mol; Mw = 6140 g/mol; OH-Zahl: 70 mg KOH/g Polymer; Säurezahl: 169 mg KOH/g Polymer) wurde in Form einer gelb gefärbten, hoch viskosen Flüssigkeit erhalten, welche nach Neutralisierung von 100% der terminalen Carbonsäuregruppen mit Triethanolamin sehr gut wasserlöslich war. Das Polymer A.3 wurde in dieser vollständig neutralisierten Form für die weiteren Versuche eingesetzt.

### Synthesebeispiel 4: Hyperverzweigter Polyester A.4

In einem Vierhalskolben, der mit Rührer, Innenthermometer, Stickstoffeinleitungsrohr und absteigendem Kühler mit Auffanggefäß versehen war, wurden unter Begasung mit trockenem Stickstoff 371,9 g des trifunktionellen Alkohols TMP x 12,2 EO und 230,7 g Alkenylbernsteinsäureanhydrid (Pentasize 8) vorgelegt, mit 0,3 g DBTL versetzt und das Reaktionsgemisch unter Rühren auf 180-200 °C erhitzt. Nach einer Reaktionszeit von 7 h wurde die Reaktion durch Abkühlen auf Raumtemperatur beendet. Das Polymer A.4 (Mn = 3850 g/mol; Mw = 38070 g/mol; OH-Zahl: 73 mg KOH/g Polymer; Säurezahl: 27 mg KOH/g Polymer) wurde in Form einer gelb gefärbten, hoch viskosen Flüssigkeit erhalten, welche bereits ohne Neutralisierung der terminalen Carbonsäuregruppen sehr gut wasserlöslich war. Das Polymer A.4 wurde in dieser vollständig protonierten Form für die weiteren Versuche eingesetzt.

### Synthesebeispiel 5: Hyperverzweigter Polyester A.5

In einem Vierhalskolben, der mit Rührer, Innenthermometer, Stickstoffeinleitungsrohr und absteigendem Kühler mit Auffanggefäß versehen war, wurden unter Begasung mit trockenem Stickstoff 228,8 g des trifunktionellen Alkohols TMP x 12,2 EO, 34,1 g Polyethylenglykolmonomethylether (M = 500 g/mol), 204,9 g PIBSA 1000 und 32,2 g Bernsteinsäure vorgelegt, mit 0,2 g DBTL versetzt und das Reaktionsgemisch unter Rühren auf 160-180 °C erhitzt. Nach 2 h wurde die Reaktion durch Abkühlen auf Raumtemperatur beendet. Das Polymer A.5 (Mn = 1540 g/mol; Mw = 5200 g/mol; OH-Zahl: 65 mg KOH/g Polymer; Säurezahl: 32 mg KOH/g Polymer) wurde in Form einer gelb gefärbten, hoch viskosen Flüssigkeit erhalten, welche bereits ohne Neutralisierung der terminalen Carbonsäuregruppen sehr gut wasserlöslich war. Das Polymer A.5 wurde in dieser vollständig protonierten Form für die weiteren Versuche eingesetzt.

### Synthesebeispiel 6: Hyperverzweigter Polyester A.6

In einem Vierhalskolben, der mit Rührer, Innenthermometer, Stickstoffeinleitungsrohr und absteigendem Kühler mit Auffanggefäß versehen war, wurden unter Begasung mit trockenem Stickstoff 253,8 g des trifunktionellen Alkohols TMP x 12,2 EO, 227,3 g PIBSA 1000 und 19,0 g Bernsteinsäureanhydrid vorgelegt, mit 0,2 g DBTL versetzt und das Reaktionsgemisch unter Rühren auf 180 -200 °C erhitzt. Nach 3,5 h wurde die Reaktion durch Abkühlen auf Raumtemperatur beendet. Das Polymer A.6 (Mn = 2700 g/mol; Mw = 7000 g/mol; OH-Zahl: n.b.; Säurezahl: 22 mg KOH/g Polymer) wurde in Form einer gelb gefärbten, hoch viskosen Flüssigkeit erhalten, welche bereits ohne Neutralisierung der terminalen Carbonsäuregruppen sehr gut wasserlöslich war. Das Polymer A.6 wurde in dieser vollständig protonierten Form für die weiteren Versuche eingesetzt.

### Synthesebeispiel 7: Hyperverzweigter Polyester A.7

In einem Vierhalskolben, der mit Rührer, Innenthermometer, Stickstoffeinleitungsrohr und absteigendem Kühler mit Auffanggefäß versehen war, wurden unter Begasung mit trockenem Stickstoff 270,11 g des trifunktionellen Alkohols TMP x 3,2 EO, 282,8 g Sebacinsäure und 245,1 g Alkenylbernsteinsäureanhydrid (Pentasize 8) vorgelegt, mit 0,2 g DBTL versetzt und das Reaktionsgemisch unter Rühren auf 160-170 °C erhitzt. Nach 3,5 h und einer abgeschiedenen Wassermenge von 20 mL (Umsatz 26%) wurde die Reaktion durch Abkühlen auf Raumtemperatur beendet. Das Polymer A.7 (Mn = 1240 g/mol; Mw = 8290 g/mol; OH-Zahl: 52 mg KOH/g Polymer; Säurezahl: 134 mg KOH/g Polymer) wurde in Form einer gelb gefärbten, hoch viskosen Flüssigkeit erhalten, welche nach Neutralisierung von 75% der terminalen Carbonsäuregruppen mit Triethanolamin sehr gut wasserlöslich war. Das Polymer A.7 wurde in dieser teilneutralisierten Form für die weiteren Versuche eingesetzt.

### Synthesebeispiel 8: Hyperverzweigter Polyester mit erhöhter Säurezahl A.8

In einem Vierhalskolben, der mit Rührer, Innenthermometer, Stickstoffeinleitungsrohr und absteigendem Kühler mit Auffanggefäß versehen war, wurden unter Begasung mit trockenem Stickstoff 194,6 g des trifunktionellen Alkohols TMP x 3,2 EO und 305,5 g Sebacinsäure vorgelegt, mit 0,2 g DBTL versetzt und das Reaktionsgemisch unter Rühren auf 160-170 °C erhitzt. Nach 2 h und einer abgeschiedenen Wassermenge von 26 mL (Umsatz 47%) wurde auf Raumtemperatur abgekühlt. Von dem so erhaltenen Produkt wurden 102,6 g mit 2,7 g Bernsteinsäureanhydrid versetzt und auf 130 °C erhitzt. Nach 2 h wurde auf 60 °C abgekühlt und das im Kühler sublimierte Bernsteinsäureanhydrid durch Zugabe von 50 g Aceton wieder der Reaktion zurückgeführt. Unter Abdestillieren des Acetons wurde die Reaktionsmischung während 3 h langsam bis auf 110 °C erhitzt. Dann wurde die Reaktion durch Abkühlen auf Raumtemperatur beendet. Das Polymer A.8 (Mn = 1190 g/mol; Mw = 46650 g/mol; OH-Zahl: 27 mg KOH/g Polymer; Säurezahl: 154 mg KOH/g Polymer) wurde in Form einer gelb gefärbten, hoch viskosen Flüssigkeit erhalten, welche nach Neutralisierung von 100% der terminalen Carbonsäuregruppen mit Triethanolamin sehr gut wasserlöslich war. Das Polymer A.8 wurde in dieser vollständig neutralisierten Form für die weiteren Versuche eingesetzt.

### Synthesebeispiel 9: Hyperverzweigter Polyester mit erhöhter Säurezahl A.9

In einem Vierhalskolben, der mit Rührer, Innenthermometer, Stickstoffeinleitungsrohr und absteigendem Kühler mit Auffanggefäß versehen war, wurden unter Begasung mit trockenem Stickstoff 1290,0 g des trifunktionellen Alkohols TMP x 5,2 PO und 1047,0, g Alkenylbernsteinsäureanhydrid (Pentasize 8) vorgelegt, mit 0,2 g DBTL versetzt. Das Reaktionsgemisch wurde unter Rühren auf 160-180 °C erhitzt und bei einem Vakuum von 50 mbar für 13 h bei dieser Temperatur gerührt. Dann wurde die Reaktion durch Abkühlen auf Raumtemperatur beendet. Von dem so erhaltenen Produkt wurden 536,7 g mit 70,1 g Bernsteinsäureanhydrid versetzt und auf 130 °C erhitzt. Nach 2 h wurde auf 60 °C abgekühlt und das im Kühler sublimierte Bernsteinsäureanhydrid durch Zugabe von 45 g Aceton wieder der Reaktion zurückgeführt. Unter Abdestillieren des Acetons wurde die Reaktionsmischung während 1 h langsam bis auf 130 °C erhitzt. Dann wurde auf Raumtemperatur abgekühlt. Das Polymer A.9 (Mn = 2430 g/mol; Mw = 9170 g/mol; OH-Zahl: 18 mg KOH/g Polymer; Säurezahl: 92 mg KOH/g Polymer) wurde in Form einer gelb gefärbten, hoch viskosen Flüssigkeit erhalten, welche nach Neutralisierung von 50% der terminalen Carbonsäuregruppen mit Triethanolamin sehr gut wasserlöslich war. Das Polymer A.9 wurde in dieser teilneutralisierten Form für die weiteren Versuche eingesetzt.

### Synthesebeispiel 10: Hyperverzweigter Polyester mit erhöhter Säurezahl A.10

In einem Vierhalskolben, der mit Rührer, Innenthermometer, Stickstoffeinleitungsrohr und absteigendem Kühler mit Auffanggefäß versehen war, wurden unter Begasung mit trockenem Stickstoff 270,11 g des trifunktionellen Alkohols TMP x 3,2 EO, 282,8 g Sebacinsäure und 245,1, g Alkenylbernsteinsäureanhydrid (Pentasize 8) vorgelegt, mit 0,2 g DBTL versetzt und das Reaktionsgemisch unter Rühren auf 160-170 °C erhitzt. Nach einer Reaktionszeit von 3,5 h und einer abgeschiedenen Wassermenge von 20 mL (Umsatz 26%) wurde die Reaktion durch Abkühlen auf Raumtemperatur beendet. Von dem so erhaltenen Produkt wurden 209,6 g mit 5,8 g Bernsteinsäureanhydrid versetzt und auf 130 °C erhitzt. Nach 2 h wurde abgekühlt und das im Kühler sublimierte Bernsteinsäureanhydrid durch Zugabe von 42 g Aceton wieder der Reaktion zurückgeführt. Unter Abdestillieren des Acetons wurde die Reaktionsmischung während 1 h langsam auf 100 °C erhitzt. Dann wurde auf Raumtemperatur abgekühlt. Das Polymer A.10 (Mn = 1240 g/mol; Mw = 14120 g/mol; OH-Zahl: 34 mg KOH/g Polymer; Säurezahl: 147 mg KOH/g Polymer) wurde in Form einer gelb gefärbten, hoch viskosen Flüssigkeit erhalten, welche nach Neutralisierung von 75% der terminalen Carbonsäuregruppen mit Triethanolamin sehr gut wasserlöslich war. Das Polymer A.10 wurde in dieser teilneutralisierten Form für die weiteren Versuche eingesetzt.

### Synthesebeispiel 11: Hyperverzweigter Polyester-Kern (A.11a) mit einer Schale aus linearen PEG-Ketten, Funktionalisierungsgrad 100%, A.11

Stufe 1 a: Herstellung eines hyperverzweigten Polyesters A.11 a
   In einem Vierhalskolben, der mit Rührer, Innenthermometer, Stickstoffeinleitungsrohr und absteigendem Kühler mit Auffanggefäß versehen war, wurden unter Begasung mit trockenem Stickstoff 251,7 g des trifunktionellen Alkohols TMP x 5,2 PO und 248,3 g Sebacinsäure vorgelegt, mit 0,3 g Katalysator DBTL versetzt und das Reaktionsgemisch unter Rühren auf 160-180 °C erhitzt. Nach einer Reaktionszeit von 8,5 h und einer abgeschiedenen Wassermenge von 16 mL (Umsatz 36%) wurde die Reaktion durch Abkühlen auf Raumtemperatur beendet. Das Polymer A.11 a (Mn = 1110 g/mol; Mw = 9990 g/mol; OH-Zahl: 49 mg KOH/g Polymer; Säurezahl: 124 mg KOH/g Polymer) wurde in Form einer gelb gefärbten, hoch viskosen Flüssigkeit erhalten, welche nicht wasserlöslich war.
Stufe 1 b (A.11 b): 70,0 g Polyethylenglykolmonomethylether (Mn = 750 g/mol) wurden in einem 250 mL Dreihalskolben, bestückt mit einem Rückflusskühler und einem Innenthermometer, vorgelegt und im Vakuum bei 80 °C von Wasserresten befreit. Nach dem Abkühlen auf Raumtemperatur wurde der Ansatz unter Stickstoff gesetzt und das Polymer in 70,0 g n-Butylacetat gelöst. Nun wurden 20,2 g Isophorondiisocyanat (IPDI) zugegeben und das Gemisch auf 50 °C erhitzt. Durch Zugabe von 11 mg Zink-neodecanoat (Tegokat 616, TIB Chemicals, Mannheim), gelöst in 1 mL n-Butylacetat, wurde die Reaktion gestartet und imVerlauf von ca. 12 h bei 50 °C bis auf einen NCO-Gehalt von 2,12% gefahren. Anschließend wurde die Reaktion durch Abkühlen auf -20 °C beendet. Das Umsetzungsprodukt A.11 b wurde ohne weitere Aufarbeitung direkt in Stufe 2 eingesetzt.
Stufe 2 (A.11): 25,0 g des hyperverzweigten Polyester-Kerns A.11a wurden in einem 250 mL Dreihalskolben, bestückt mit einem Rückflusskühler und einem Innenthermometer, in 10 g Tetrahydrofuran gelöst und unter Stickstoff mit 51,0 g des Reaktionsgemischs A.11 b versetzt. Nun wurde der Ansatz auf 60 °C erhitzt und die Reaktion durch Zugabe von 5 mg DBTL, gelöst in 1 mL n-Butylacetat, gestartet. Nach dem vollständigen Umsatz aller NCO-Gruppen (NCO-Gehalt 0%) wurde der Ansatz abgekühlt und das Lösungsmittel im Vakuum entfernt. Schließlich wurde das linear-dendritische Co-polymer A.11 (Mn = 1860 g/mol) in Form einer gelb gefärbten, hoch viskosen Flüssigkeit erhalten, welche vollständig wasserlöslich war.

### Synthesebeispiel 12: Hyperverzweigter Polyester-Kern (A.12a) mit einer Schale aus linearen PEG-Ketten, Funktionalisierungsgrad 50%, A.12

Stufe 1 a: Herstellung eines hyperverzweigten Polyesters, A.12a
   In einem Vierhalskolben, der mit Rührer, Innenthermometer, Stickstoffeinleitungsrohr und absteigendem Kühler mit Auffanggefäß versehen war, wurden unter Begasung mit trockenem Stickstoff 200,0 g Trimethylolpropan und 300,7 g Sebacinsäure vorgelegt, mit 0,1 g Katalysator DBTL versetzt und das Reaktionsgemisch unter Rühren auf 160-180 °C erhitzt. Nach einer Reaktionszeit von 105 min und einer abgeschiedenen Wassermenge von 39 mL (Umsatz 72%) wurde die Reaktion durch Abkühlen auf Raumtemperatur beendet. Das Polymer A.12a (Mn = 1410 g/mol; Mw = 34520 g/mol; OH-Zahl: 210 mg KOH/g Polymer; Säurezahl: 40 mg KOH/g Polymer) wurde in Form einer gelb gefärbten, hoch viskosen Flüssigkeit erhalten, welche nicht wasserlöslich war.
Stufe 1 b (A.12b): 280,0 g Polyethylenglykolmonomethylether (Mn = 500 g/mol) wurden in einem 1000 mL Dreihalskolben, bestückt mit einem Rückflusskühler und einem Innenthermometer, vorgelegt und im Vakuum bei 80 °C von Wasserresten befreit. Nach dem Abkühlen auf Raumtemperatur wurde der Ansatz unter Stickstoff gesetzt und das Polymer in 280,0 g n-Butylacetat gelöst. Nun wurden 111,0 g IPDI zugegeben und das Gemisch auf 50 °C erhitzt. Durch Zugabe von 42 mg Zink-neodecanoat, gelöst in 1 mL n-Butylacetat, wurde die Reaktion gestartet und imVerlauf von 5,5 h bei 50 °C bis auf einen NCO-Gehalt von 2,79% gefahren. Anschließend wurde die Reaktion durch Abkühlen auf -20 °C beendet. Das Umsetzungsprodukt A.12b wurde ohne weitere Aufarbeitung direkt in Stufe 2 eingesetzt.
Stufe 2 (A.12): 18,0 g des hydrophoben hyperverzweigten Polyester-Kerns A.12a wurden in einem 250 mL Dreihalskolben, bestückt mit einem Rückflusskühler und einem Innenthermometer, vorgelegt und unter Stickstoff mit 50,6 g des Reaktionsgemischs A.12b versetzt. Nun wurde der Ansatz auf 80 °C erhitzt und die Reaktion durch Zugabe von 5 mg DBTL, gelöst in 1 mL n-Butylacetat, gestartet. Nach dem vollständigen Umsatz aller NCO-Gruppen (NCO-Gehalt 0%) wurde der Ansatz abgekühlt und das Lösungsmittel im Vakuum entfernt. Schließlich wurde das linear-dendritische Copolymer A.12 (Mn = 2700 g/mol) in Form einer gelb gefärbten, hoch viskosen Flüssigkeit erhalten, welche vollständig wasserlöslich war.

### Synthesebeispiel 13: Hyperverzweigter Polyester-Kern (A.13a) mit einer Schale aus linearen PEG-Ketten, Funktionalisierungsgrad 100%, A.13

Stufe 1 a: Herstellung eines hyperverzweigten Polyesters, A.13a
   In einem Vierhalskolben, der mit Rührer, Innenthermometer, Stickstoffeinleitungsrohr und absteigendem Kühler mit Auffanggefäß versehen war, wurden unter Begasung mit trockenem Stickstoff 222,9 g des trifunktionellen Alkohols TMP x 3,2 EO und 277,3 g Pentasize 68 vorgelegt, mit 0,2 g DBTL versetzt und das Reaktionsgemisch unter Rühren auf 200 °C erhitzt. Nach einer Reaktionszeit von 6 h wurde die Reaktion durch Abkühlen auf Raumtemperatur beendet. Das Polymer A.13a (Mn = 1990 g/mol; Mw = 123860 g/mol; OH-Zahl: 41 mg KOH/g Polymer; Säurezahl: 18 mg KOH/g Polymer) wurde in Form einer gelb gefärbten, hoch viskosen Flüssigkeit erhalten, welche nicht wasserlöslich war.
Stufe 1 b (A.13b): 50,0 g Polyethylenglykolmonomethylether (Mn = 1000 g/mol) wurden in einem 250 mL Dreihalskolben, bestückt mit einem Rückflusskühler und einem Innenthermometer, vorgelegt und im Vakuum bei 80 °C von Wasserresten befreit. Nach dem Abkühlen auf Raumtemperatur wurde der Ansatz unter Stickstoff gesetzt und das Polymer in 50,0 g n-Butylacetat gelöst. Nun wurden 10,1 g Isophorondiisocyanat (IPDI) zugegeben und das Gemisch auf 50 °C erhitzt. Durch Zugabe von 8 mg Zink-neodecanoat gelöst in 1 mL n-Butylacetat, wurde die Reaktion gestartet und imVerlauf von 4,5 h bei 50 °C bis auf einen NCO-Gehalt von 1,43% gefahren. Anschließend wurde die Reaktion durch Abkühlen auf -20 °C beendet. Das Umsetzungsprodukt A.13b wurde ohne weitere Aufarbeitung direkt in Stufe 2 eingesetzt.
Stufe 2 (A.13): 6,0 g von A.13a wurden in einem 250 mL Dreihalskolben, bestückt mit einem Rückflusskühler und einem Innenthermometer, in 54,0 g n-Butylacetat gelöst und unter Stickstoff mit 12,9 g des Reaktionsgemischs A.13b versetzt. Nun wurde der Ansatz auf 80 °C erhitzt und die Reaktion durch Zugabe von 2 mg DBTL, gelöst in 1 mL n-Butylacetat, gestartet. Nach dem vollständigen Umsatz aller NCO-Gruppen (NCO-Gehalt 0%) wurde der Ansatz abgekühlt und das Lösungsmittel im Vakuum entfernt. Schließlich wurde das linear-dendritische Copolymer A.13 (Mn = 3390 g/mol) in Form einer gelb gefärbten, hoch viskosen Flüssigkeit erhalten, welche vollständig wasserlöslich war.

### Synthesebeispiel 14: Hyperverzweigter Polyester-Kern (A.14a) mit einer Schale aus linearen PEG-Ketten, Funktionalisierungsgrad 100%, A.14

Stufe 1a: Herstellung eines hyperverzweigten Polyesters, A.14a
   In einem Vierhalskolben, der mit Rührer, Innenthermometer, Stickstoffeinleitungsrohr und absteigendem Kühler mit Auffanggefäß versehen war, wurden unter Begasung mit trockenem Stickstoff 70,3 g des trifunktionellen Alkohols TMP x 1,1 PO, 84,1 g Sebacinsäure und 347,2 g PIBSA 1000 vorgelegt, mit 0,1 g Katalysator DBTL versetzt und das Reaktionsgemisch unter Rühren auf 160-165 °C erhitzt. Nach einer Reaktionszeit von 2,5 h wurde die Reaktion durch Abkühlen auf Raumtemperatur beendet. Das Polymer A.14a (Mn = 1450 g/mol; Mw = 4750 g/mol; OH-Zahl: 56 mg KOH/g Polymer; Säurezahl: 68 mg KOH/g Polymer) wurde in Form einer gelb gefärbten, hoch viskosen Flüssigkeit erhalten, welche nicht wasserlöslich war.
Stufe 1 b (A.14b): 61,8 g Polyethylenglykolmonomethylether (Mn = 500 g/mol) wurden in einem 250 mL Dreihalskolben, bestückt mit einem Rückflusskühler und einem Innenthermometer, vorgelegt und im Vakuum bei 80 °C von Wasserresten befreit. Nach dem Abkühlen auf Raumtemperatur wurde der Ansatz unter Stickstoff gesetzt und das Polymer in 61,8 g n-Butylacetat gelöst. Nun wurden 25,0 g Isophorondiisocyanat (IPDI) zugegeben und das Gemisch auf 50 °C erhitzt. Durch Zugabe von 10 mg Zink-neodecanoat gelöst in 1 mL n-Butylacetat, wurde die Reaktion gestartet und imVerlauf von 4,5 h bei 50 °C bis auf einen NCO-Gehalt von 2,84% gefahren. Anschließend wurde die Reaktion durch Abkühlen auf -20 °C beendet. Das Umsetzungsprodukt A.14b wurde ohne weitere Aufarbeitung direkt in Stufe 2 eingesetzt.
Stufe 2 (A.14): 7,0 g des hydrophoben hyperverzweigten Polyester-Kerns A.14a wurden in einem 500 mL Dreihalskolben, bestückt mit einem Rückflusskühler und einem Innenthermometer, in 7,0 g n-Butylacetat gelöst und unter Stickstoff mit 12,1 g des Reaktionsgemischs A.14b versetzt. Nun wurde der Ansatz auf 80 °C erhitzt und die Reaktion durch Zugabe von 2 mg DBTL, gelöst in 1 mL n-Butylacetat, gestartet. Nach dem vollständigen Umsatz aller NCO-Gruppen (NCO-Gehalt 0%) wurde der Ansatz abgekühlt und das Lösungsmittel im Vakuum entfernt. Schließlich wurde das linear-dendritische Copolymer A.14 (Mn = 2200 g/mol) in Form einer gelb gefärbten, hoch viskosen Flüssigkeit erhalten, welche vollständig wasserlöslich war.

### Vergleichsbeispiel 15: Hyperverzweigter Polyester A.15 (nicht erfindungsgemäß)

In einem Vierhalskolben, der mit Rührer, Innenthermometer, Stickstoffeinleitungsrohr und absteigendem Kühler mit Auffanggefäß versehen war, wurden unter Begasung mit trockenem Stickstoff 92,1 g des trifunktionellen Alkohols Glycerin und 142,0 g Bernsteinsäure vorgelegt, mit 0,1 g Katalysator DBTL versetzt und das Reaktionsgemisch unter Rühren auf 160-180 °C erhitzt. Nach einer Reaktionszeit von 2,5 h und einer abgeschiedenen Wassermenge von 30 mL (Umsatz 68%) wurde auf 140 °C abgekühlt und dem Reaktionsgemisch weitere 50,6 g des trifunktionellen Alkohols Glycerin zugegeben. Anschließend wurde unter Rühren auf 190 °C erhitzt. Nach einer Reaktionszeit von weiteren 4h wurde für den Zeitraum von 1 h ein Vakuum von 400 mbar angelegt. Dann wurde die Reaktion durch Abkühlen auf Raumtemperatur beendet. Das Polymer A.15 (Mn = 660 g/mol; Mw = 2050 g/mol; OH-Zahl: 536 mg KOH/g Polymer; Säurezahl: 24 mg KOH/g Polymer) wurde in Form einer gelb gefärbten, hoch viskosen Flüssigkeit erhalten, welche nach Neutralisierung von 50% der terminalen Carbonsäuregruppen mit Triethanolamin sehr gut wasserlöslich war. Das Polymer A.15 wurde in dieser teilneutralisierten Form für die weiteren Versuche eingesetzt.

### Vergleichsbeispiel 16: Hyperverzweigter Polyester A.16 (nicht erfindungsgemäß)

In einem Vierhalskolben, der mit Rührer, Innenthermometer, Stickstoffeinleitungsrohr und absteigendem Kühler mit Auffanggefäß versehen war, wurden unter Begasung mit trockenem Stickstoff 152,1 g Trimethylolpropan und 348,9 g Adipinsäure vorgelegt und das Reaktionsgemisch unter Rühren auf 160-170 °C erhitzt. Nach einer Reaktionszeit von 70 min und einer abgeschiedenen Wassermenge von 44 mL (Umsatz 51 %) wurde die Reaktion durch Abkühlen auf Raumtemperatur beendet. Das Polymer A.16 (Mn = 710 g/mol; Mw = 75160 g/mol; OH-Zahl: n. b.; Säurezahl: 134 mg KOH/g Polymer) wurde in Form einer gelb gefärbten, hoch viskosen Flüssigkeit erhalten, welche nach Neutralisierung von 100% der terminalen Carbonsäuregruppen mit Triethanolamin sehr gut wasserlöslich war. Das Polymer A.16 wurde in dieser vollständig neutralisierten Form für die weiteren Versuche eingesetzt.

### Beispiel 17: Herstellung und Redispergierung einer Festformulierung ("Solid Solution")

In einem Becherglas wurde der entsprechende hyperverzweigte Polyester in Isopropanol gelöst und die Lösung nachfolgend mit dem Fungizid Pyraclostrobin versetzt. Die Gesamtkonzentration der so hergestellten, homogenen Polymer/Wirkstoff-Mischung in Isopropanol betrug 20 Gew%, wobei stets ein Polymer/Wirkstoffverhältnis von 3:1 eingestellt wurde. Nun wurde die Lösung auf kleinen Aluminiumplatten ausgegossen, so dass nach dem Entfernen des organischen Lösungsmittels im Vakuum (<100 mbar, 50 °C, 24 h) dünne Filme (<1 mm) erhalten werden konnten. Mit Hilfe kleiner Mengen an getrocknetem Filmmaterial wurde versucht, 1 Gew%ige Lösungen durch Redispergierung in destilliertem Wasser herzustellen. Falls erfolgreich, so wurde die Polymer/Wirkstoff-Dispersion in Wasser hinsichtlich ihrer Qualität (Trübung, Bildung von Bodensatz) beurteilt. Die Daten zu den hergestellten Festformulierungen sind in Tabelle 1 zusammengestellt.

**Tabelle 1**

| Hyperverzweigter Polyester | optische Beurteilung des Films nach Herstellung | optische Beurteilung nach Redispergierung |
|---|---|---|
| A.1 | klar | klar |
| A.9 | klar | klar |
| A.10 | klar | klar |

### Beispiel 18 - Solubilisierung von Piroxicam, Carbamazepin, Estradiol und Clotrimazol

In einem 50 mL Becherglas wurden 2 g Polymer eingewogen. Anschließend wurde dem Ansatz jeweils 0,2 g Wirkstoff zugewogen, um eine übersättigte Lösung zu erhalten. Nun wurde soviel Phosphatpuffer pH 7,0 zugegeben, dass ein Masseverhältnis Polymer:Phosphatpuffer von 1:9 vorlag. Das Gemisch wurde nun 72 h bei Raumtemperatur mit Hilfe eines Magnetrührers gerührt. Nach einstündiger Ruhezeit wurde überschüssiger (d.h. nicht solubilisierter) Wirkstoff durch Filtration abgetrennt. Die so erhaltene, klare bzw. opake Lösung wurde anschließend auf ihren Wirkstoffgehalt mittels UV-Spektroskopie oder HPLC untersucht. Die Wellenlängen der UV-spektroskopischen Messungen (falls anwendbar) sind in Tabelle 2 zusammengestellt. Die Ergebnisse der Solubilisierungsversuche sind in Tabelle 3 zusammengestellt.

**Tabelle 2**

| Wirkstoff | Wellenlänge der UV-Messung [nm] |
|---|---|
| Piroxicam | 356 |
| Carbamazepin | 286 |
| Estradiol | 282 |
| Clotrimazol | HPLC |

**Tabelle 3: Löslichkeit von Wirkstoffen [mg/l] in Gegenwart von hyperverzweigtem Polyester**

| Polyester | Piroxicam | Carbamazepin | Estradiol | Clotrimazol |
|---|---|---|---|---|
| ohne | 420 | 140 | <100 | <100 |
| A.1 ^{a)} | 6200 | 1700 | 1100 | 4700 |
| A.6 | n.b. ^{b)} | 550 | 3350 | 1390 |
| A.9 | 1300 | 2700 | 300 | 5500 |
| A.10 | 4800 | 1800 | 900 | 5600 |

| | | | | |
|---|---|---|---|---|
| a) Neutralisationsgrad der Carbonsäuregruppen betrug in diesem Test 100%. b) n.b. = nicht bestimmt | | | | |

### Beispiel 19 - Solubilisierung von Pyren, Pyraclostrobin und Fipronil

In einem 50 mL Becherglas wurden 100 mg Polymer eingewogen und in 9,900 g dest. Wasser gelöst. Anschließend wurde dem Ansatz jeweils 100 mg Wirkstoff zugewogen, um eine übersättigte Lösung zu erhalten. Das Gemisch wurde nun 24 h bei Raumtemperatur mit Hilfe eines Magnetrührers gerührt. Nach einstündiger Ruhezeit wurde überschüssiger (d.h. nicht solubilisierter) Wirkstoff durch Zentrifugieren abgetrennt. Die so erhaltene, klare bzw. opake Lösung wurde anschließend auf ihren Wirkstoffgehalt mittels UV-Spektroskopie untersucht. Die Wellenlängen der UV-spektroskopischen Messungen sind in Tabelle 4 zusammengestellt. Die Ergebnisse der Solubilisierungsversuche sind in Tabelle 5 zusammengestellt.

**Tabelle 4**

| Wirkstoff | Wellenlänge der UV-Messung [nm] |
|---|---|
| Pyren | 334 |
| Pyraclostrobin | 277 |
| Fipronil | 280 |

**Tabelle 5: Löslichkeit von Wirkstoffen [mg/l] in Gegenwart von hyperverzweigtem Polyester**

| Polymer | Pyren | Pyraclostrobin | Fipronil |
|---|---|---|---|
| ohne | 0,1 | 22,5 | 3 |
| A.1 | 234 | 619 | 104 |
| A.2 | 189 | 531 | n.b. |
| A.3 | 169 | n.b. | 144 |
| A.4 | 224 | 578 | 174 |
| A.5 | 81 | n.b. | 174 |
| A.6 | 103 | n.b. | 198 |
| A.7 | 247 | 558 | n.b. |
| A.8 | 227 | 630 | n.b. |
| A.9 | 248 | 579 | 141 |
| A.10 | 281 | 736 | 147 |
| A.11 | 200 | n.b. | 227 |
| A.12 | 180 | 554 | 290 |
| A.13 | 160 | n.b. | 109 |
| A.14 | 80 | n.b. | 74 |

| | | | |
|---|---|---|---|
| n.b. = nicht bestimmt | | | |

### Beispiel 20 - Vergleichsversuche zur Solubilisierung mit nicht erfindungsgemäßen hyperverzweigten Polyestern

Die hyperverzweigten Polyester a.1 bis a.5 und c.1 wurden hergestellt wie in WO 2007/125028 beschrieben in Beispiel I.1 bis I.5 und II.2. Zusätzlich wurden die oben beschriebenen hyperverzweigten Polyester A.15 und A.16 getestet. Alle Polymere wurden nach der oben beschriebenen Arbeitsvorschrift (siehe Beispiel 19) auf ihre Solubilisierung hin getestet. Die Ergebnisse sind in Tabelle 6 zusammengestellt.

**Tabelle 6: Solubilisierung mit nicht erfindungsgemäßen hyperverzweigten Polyestern**

| Polymer | Bausteine | Pyren | Fipronil |
|---|---|---|---|
| a.1 | Bernsteinsäure + 12EO-TMP | 0,1 | n.b. |
| a.2 | Bernsteinsäure + 5EO-Glycerin | 0,1 | n.b. |
| a.3 | Adipinsäure + 12EO-TMP | 0,3 | n.b. |
| a.4 | Adipinsäure + 5EO-Glycerin | 1,2 | n.b. |
| a.5 | Bernsteinsäure + Glycerin | 0,1 | n.b. |
| c.1 | Adipinsäure + Glycerin, verknüpft mit MPEG750 | 14 | n.b. |
| A.15 | Bernsteinsäure + Glycerin | 2 | 11 |
| A.16 | Adipinsäure + TMP | 45 | n.b. |

| | | | |
|---|---|---|---|
| n.b. = nicht bestimmt. TMP: Trimethylolpropan; 12EO-TMP: TMP ethoxyliert mit 12 mol EO pro mol TMP; 5EO-Glycerin: Glycerin ethoxyliert mit 5 mol EO pro mol Glycerin; MPEG750: Monomethylpolyethylenglykol, Molmasse 750 g/mol. | | | |

Der direkte Vergleich der Polymere (a.1)-(a.5) und A.15/A.16 (Tabelle 6) mit den strukturell ähnlichen, aber auf Sebacinsäure und/oder ASA und/oder PIBSA basierenden, erfindungsgemäßen Polymeren A.1-A.10 (Tabelle 5) zeigt, dass die erfindungsgemäßen Polymere signifikant höhrere Solubilisierungskapazitäten aufweisen (1-3 Zehnerpotenzen höher).

Der direkte Vergleich des linear-dendritischen (PEG-funktionalisierten, hyperverzweigten) Polyesters (c.1) (Tabelle 6) mit den strukturell ähnlichen, aber auf Sebacinsäure und/oder ASA und/oder PIBSA basierenden, erfindungsgemäßen linear-dendritischen Polyestern A.11-A.14 (Tabelle 5) zeigt, dass auch hier die erfindungsgemäßen Polymere signifikant höhrere Solubilisierungskapazitäten aufweisen (ca. 2 Zehnerpotenzen).

## Patentansprüche

1. Zusammensetzung enthaltend einen Wirkstoff, der in Wasser bei 20 °C zu höchstens 10 g/L löslich ist, und einen hyperzverzweigten Polyester basierend auf einer hydrophoben Dicarbonsäure und einem mindestens trifunktionellen Alkohol, wobei
die hydrophobe Dicarbonsäure eine aliphatische C₁₀-C₃₂ Dicarbonsäure, eine Polyisobutylengruppe aufweisende Dicarbonsäure und/oder eine C₃-C₄₀ Gruppe aufweisende Bernsteinsäureeinheit Ist,
der mindestens trifunktionelle Alkohol Trimethylolethan, Trimethylolpropan, Bis(trimethylolpropan), Pentaerythrit, oder ein alkoxyliertes Derivat von Glycerin, Trimethylolethan, Trimethylolpropan, Bis(trimethylolpropan), Pentaerythrit ist, und wobei der hyperverzweigte Polyester verknüpft ist mit einer Polyalkylenoxidgruppe und/oder einer funktionalen C₁-C₂₄ Endgruppe enthaltend eine Säuregruppe oder zwei Alkoholgruppen.

2. Zusammensetzung nach Anspruch 1, wobei die funktionale C₁-C₂₄ Endgruppe ein Bernsteinsäurederivat ist.

3. Zusammensetzung nach einem der Ansprüche 1 bis 2, wobei die C₁₀-C₃₂ Dicarbonsäure Sebacinsäure ist.

4. Zusammensetzung nach einem der Ansprüche 1 bis 3, wobei die hydrophobe Dicarbonsäure eine Polyisobutylengruppe aufweisende Dicarbonsäure und/oder eine C₃-C₄₀ Gruppe aufweisende Bernsteinsäureeinheit ist.

5. Zusammensetzung nach einem der Ansprüche 1 bis 4, wobei das alkoxilierte Derivat von Glycerin, Trimethylolethan, Trimethylolpropan, Bis(trimethylolpropan) oder Pentaerythrit mit 1,1 bis 20 Ethylenoxid- und/oder Propylenoxideinheiten alkoxyliert ist.

6. Zusammensetzung nach einem der Ansprüche 1 bis 5, wobei der hyperverzweigte Polyester mit der Polyalkylenoxidgruppe mittels eines Polyisocyanat-Linkers verknüpft ist.

7. Zusammensetzung nach Anspruch 6, wobei der Polyisocyanat-Linker ausgewählt ist aus Toluol-2,4-diisocyanat, Toluol-2,6-diisocyanat, 4'-Diphenylmethandiisocyanat, cis- und trans-Isophorondiisocyanat, oder Mischungen dieser Verbindungen.

8. Zusammensetzung nach einem der Ansprüche 1 bis 7, wobei die Polyalkylenoxidgruppe ein Polyethylenglykol oder ein Polyethylenglykolmonoalkylether mit einer Molmasse von 200 bis 10.000 g/mol ist.

9. Zusammensetzung nach einem der Ansprüche 1 bis 8, wobei die Säurezahl des hyperverzweigten Polyesters 25 bis 300 mg KOH pro Gramm Polymer beträgt.

10. Zusammensetzung nach einem der Ansprüche 1 bis 9, wobei die Hydroxyzahl des hyperverzweigten Polyesters 10 bis 500 mg KOH pro Gramm Polymer beträgt.

11. Zusammensetzung nach einem der Ansprüche 1 bis 10, wobei das Gewichtsverhältnis des hyperverzweigten Polyesters zu dem Wirkstoff im Bereich 1 : 2 bis 25 : 1 liegt.

12. Zusammensetzung nach einem der Ansprüche 1 bis 11, wobei der Wirkstoff ein agrochemischer Wirkstoff ist.

13. Hyperverzweigter Polyester gemäß einem der Ansprüche 1 bis 10, wobei der hyperverzweigte Polyester verknüpft ist mit einer Polyalkylenoxidgruppe und/oder einer funktionalen C₁-C₂₄ Endgruppe enthaltend eine Säuregruppe oder zwei Alkoholgruppen.

14. Verfahren zur Herstellung des hyperverzweigten Polyesters gemäß einem der Ansprüche 1 bis 10, indem die hydrophobe Dicarbonsäure und der trifunktionelle Alkohol polykondensiert werden, und anschließend mit einer Polyalkylenoxidgruppe und/oder einer funktionalen C₁-C₂₄ Endgruppe enthaltend eine Säuregruppe oder zwei Alkoholgruppen verknüpft werden.

15. Verwendung des hyperverzweigten Polyester gemäß einem der Ansprüche 1 bis 10 zur Solubilisierung eines Wirkstoffs, der in Wasser bei 20 °C zu höchstens 10 g/L löslich ist, in wässrigen Lösungen.

## Claims

1. A composition comprising an active ingredient with a maximum solubility in water at 20°C of 10 g/l, and a hyperbranched polyester based on a hydrophobic dicarboxylic acid and an at least trifunctional alcohol, wherein
the hydrophobic dicarboxylic acid is an aliphatic C₁₀-C₃₂ dicarboxylic acid, a dicarboxylic acid having a polyisobutylene group and/or a succinic acid unit having a C₃-C₄₀ group,
the at least trifunctional alcohol is trimethylolethane, trimethylolpropane, bis(trimethylolpropane), pentaerythritol, or an alkoxylated derivative of glycerol, trimethylolethane, trimethylolpropane, bis(trimethylolpropane), pentaerythritol, and wherein the hyperbranched polyester is joined to a polyalkylene oxide group and/or a functional C₁-C₂₄ end group comprising an acid group or two alcohol groups.

2. The composition according to claim 1, wherein the functional C₁-C₂₄ end group is a succinic acid derivative.

3. The composition according to any of claims 1 to 2, wherein the C₁₀-C₃₂ dicarboxylic acid is sebacic acid.

4. The composition according to any of claims 1 to 3, wherein the hydrophobic dicarboxylic acid is a dicarboxylic acid having a polyisobutylene group and/or a succinic acid unit having a C₃-C₄₀ group.

5. The composition according to any of claims 1 to 4, wherein the alkoxylated derivative of glycerol, trimethylolethane, trimethylolpropane, bis(trimethylolpropane) or pentaerythritol has been alkoxylated with 1.1 to 20 ethylene oxide and/or propylene oxide units.

6. The composition according to any of claims 1 to 5, wherein the hyperbranched polyester is joined to the polyalkylene oxide group by means of a polyisocyanate linker.

7. The composition according to claim 6, wherein the polyisocyanate linker is selected from toluene 2,4-diisocyanate, toluene 2,6-diisocyanate, 4'-diphenylmethane diisocyanate, cis- and trans-isophorone diisocyanate, or mixtures of these compounds.

8. The composition according to any of claims 1 to 7, wherein the polyalkylene oxide group is a polyethylene glycol or a polyethylene glycol monoalkyl ether having a molar mass of 200 to 10 000 g/mol.

9. The composition according to any of claims 1 to 8, wherein the acid number of the hyperbranched polyester is 25 to 300 mg KOH per g of polymer.

10. The composition according to any of claims 1 to 9, wherein the hydroxyl number of the hyperbranched polyester is 10 to 500 mg KOH per g of polymer.

11. The composition according to any of claims 1 to 10, wherein the weight ratio of the hyperbranched polyester to the active ingredient is in the range from 1 : 2 to 25 : 1.

12. The composition according to any of claims 1 to 11, wherein the active ingredient is an active agrochemical ingredient.

13. The hyperbranched polyester according to any of claims 1 to 10, wherein the hyperbranched polyester is joined to a polyalkylene oxide group and/or a functional C₁-C₂₄ end group comprising one acid group or two alcohol groups.

14. A process for preparing the hyperbranched polyester according to any of claims 1 to 10, by polycondensing the hydrophobic dicarboxylic acid and the trifunctional alcohol, and then joining the product to a polyalkylene oxide group and/or a functional C₁-C₂₄ end group comprising one acid group or two alcohol groups.

15. The use of the hyperbranched polyester according to any of claims 1 to 10 for solubilizing an active ingredient with a maximum solubility in water at 20°C of 10 g/l in aqueous solutions.

## Revendications

1. Composition contenant un agent actif, qui est soluble dans l'eau à 20 °C à hauteur d'au plus 10 g/l, et un polyester hyperramifié à base d'un acide dicarboxylique hydrophobe et d'un alcool au moins trifonctionnel,
l'acide dicarboxylique hydrophobe étant un acide dicarboxylique en C₁₀-C₃₂ aliphatique, un acide dicarboxylique comprenant un groupe polyisobutylène et/ou une unité acide succinique comprenant un groupe en C₃-C₄₀,
l'alcool au moins trifonctionnel étant le triméthyloléthane, le triméthylolpropane, le bis(triméthylolpropane), la pentaérythrite ou un dérivé alcoxylé de glycérine, de triméthyloléthane, de triméthylolpropane, de bis(triméthylolpropane), de pentaérythrite, et
le polyester hyperramifié étant relié avec un groupe oxyde de polyalkylène et/ou un groupe terminal en C₁-C₂₄ fonctionnel contenant un groupe acide ou deux groupes alcool.

2. Composition selon la revendication 1, dans laquelle le groupe terminal en C₁-C₂₄ fonctionnel est un dérivé de l'acide succinique.

3. Composition selon l'une quelconque des revendications 1 à 2, dans laquelle l'acide dicarboxylique en C₁₀-C₃₂ est l'acide sébacique.

4. Composition selon l'une quelconque des revendications 1 à 3, dans laquelle l'acide dicarboxylique hydrophobe est un acide dicarboxylique comprenant un groupe polyisobutylène et/ou une unité acide succinique comprenant un groupe en C₃-C₄₀.

5. Composition selon l'une quelconque des revendications 1 à 4, dans laquelle le dérivé alcoxylé de glycérine, de triméthyloléthane, de triméthylolpropane, de bis(triméthylolpropane) ou de pentaérythrite est alcoxylé avec 1,1 à 20 unités oxyde d'éthylène et/ou oxyde de propylène.

6. Composition selon l'une quelconque des revendications 1 à 5, dans laquelle le polyester hyperramifié est relié avec le groupe oxyde de polyalkylène au moyen d'un lieur polyisocyanate.

7. Composition selon la revendication 6, dans laquelle le lieur polyisocyanate est choisi parmi le 2,4-diisocyanate de toluène, le 2,6-diisocyanate de toluène, le diisocyanate de 4'-diphénylméthane, le diisocyanate de cis- et trans-isophorone ou les mélanges de ces composés.

8. Composition selon l'une quelconque des revendications 1 à 7, dans laquelle le groupe oxyde de polyalkylène est un polyéthylène glycol ou un éther monoalkylique de polyéthylène glycol ayant une masse molaire de 200 à 10 000 g/mol.

9. Composition selon l'une quelconque des revendications 1 à 8, dans laquelle l'indice d'acidité du polyester hyperramifié est de 25 à 300 mg KOH par gramme de polymère.

10. Composition selon l'une quelconque des revendications 1 à 9, dans laquelle l'indice hydroxy du polyester hyperramifié est de 10 à 500 mg KOH par gramme de polymère.

11. Composition selon l'une quelconque des revendications 1 à 10, dans laquelle le rapport en poids entre le polyester hyperramifié et l'agent actif est dans la plage allant de 1:2 à 25:1.

12. Composition selon l'une quelconque des revendications 1 à 11, dans laquelle l'agent actif est un agent actif agrochimique.

13. Polyester hyperramifié selon l'une quelconque des revendications 1 à 10, dans lequel le polyester hyperramifié est relié avec un groupe oxyde de polyalkylène et/ou un groupe terminal en C₁-C₂₄ fonctionnel contenant un groupe acide ou deux groupes alcool.

14. Procédé de fabrication du polyester hyperramifié selon l'une quelconque des revendications 1 à 10, selon lequel l'acide dicarboxylique hydrophobe et l'alcool trifonctionnel sont polycondensés, puis reliés avec un groupe oxyde de polyalkylène et/ou un groupe terminal en C₁-C₂₄ fonctionnel contenant un groupe acide ou deux groupes alcool.

15. Utilisation du polyester hyperramifié selon l'une quelconque des revendications 1 à 10 pour la solubilisation d'un agent actif, qui est soluble dans l'eau à 20 °C à hauteur d'au plus 10 g/l, dans des solutions aqueuses.
